# EUROPÄISCHE PATENTSCHRIFT

(11) **EP 1 017 703 B1**
(45) Veröffentlichungstag und Bekanntmachung des Hinweises auf die Patenterteilung: **28.04.2004**
(21) Anmeldenummer: 98952610.8
(22) Anmeldetag: 21.09.1998
(51) Int. Cl.: C07H 1/00

(54) **PENTOPYRANOSYL-NUCLEOSID, SEINE HERSTELLUNG UND VERWENDUNG**
PENTOPYRANOSYL NUCLEOSIDE, AND PRODUCTION AND USE OF THE SAME
NUCLEOSIDE DE PENTOPYRANOSYLE, SON MODE DE PREPARATION ET SON UTILISATION

(30) Priorität: 22.09.1997 DE 19741715
(43) Veröffentlichungstag der Anmeldung: 12.07.2000
(73) Patentinhaber: Nanogen Recognomics GmbH, 65926 Frankfurt am Main (DE)
(72) Erfinder: MICULKA, Christian, D-65929 Frankfurt am Main (DE); WINDHAB, Norbert, D-65795 Hattersheim (DE); BRANDSTETTER, Tilmann, D-80339 München (DE); BURDINSKI, Gerhard, D-56355 Nastätten (DE)
(74) Vertreter: Ackermann, Joachim, Dr.
(86) Internationale Anmeldenummer: PCT/EP1998/005997
(87) Internationale Veröffentlichungsnummer: WO 1999/015539

(56) Entgegenhaltungen:
- WO-A-98/25943
- S.PITSCH ET AL.: "147. Why Pentose- and Not Hexose-Nucleic Acids ? Pyranosyl-RNA ('p-RNA')" HELVETICA CHIMICA ACTA., Bd. 76, Nr. 6, 1993, Seiten 2161-2183, XP002094190 BASEL CH in der Anmeldung erwähnt
- S.PITSCH ET AL.: "122. Pyranosyl-RNA ('p-RNA'): Base-Pairing Selectivity and Potential to Replicate." HELVETICA CHIMICA ACTA., Bd. 78, Nr. 7, 1995, Seiten 1621-1635, XP002096830 BASEL CH in der Anmeldung erwähnt
- B.DOBOSZEWSKI ET AL.: "Synthesis of 3'-Deoxy-3'-C-Hydroxymethyl-aldopentopyran osyl Nucleosides and their Incorporation into Oligonucleotides. Part II." TETRAHEDRON, Bd. 51, Nr. 45, 1995, Seiten 12319-12336, XP002096831
- J.A.KELLEY ET AL.: "Furanose-pyranose Isomerization of Reduced Pyrimidine and Cyclic Urea Ribosides." JOURNAL OF MEDICINAL CHEMISTRY., Bd. 29, Nr. 11, November 1986, Seiten 2351-2358, XP002097206 WASHINGTON US
- CHEMICAL ABSTRACTS, vol. 98, no. 13, 28. März 1983 Columbus, Ohio, US; abstract no. 107682z, H.OGURA : "Glucoside Derivatives." Seite 636; Spalte 2; XP002097208 & JP 57 146796 A10. September 1982
- M.MELGUIZO ET AL.: "A New One-Step Synthesis of 8-Aminopurines Nucleoside Analogs from 6-(glycosylamino)-5-nitrosopyrimidines." JOURNAL OF ORGANIC CHEMISTRY., Bd. 57, Nr. 2, 1992, Seiten 559-565, XP002097207 EASTON US
- I.SCHLÖNVOGT ET AL.: "188. Pyranosyl-RNA ('p-RNA'): NMR and Molecular Dynamics Study of the DUplex Formed by Self-Pairing of Ribopyranosyl-(C-G-A-A-T-T-C-G)" HELVETICA CHIMICA ACTA., Bd. 79, Nr. 8, 1996, Seiten 2316-2345, XP002096829 BASEL CH
- M.BÖHRINGER ET AL.: "110. Warum Pentose- und Nicht Hexose-Nucleinsäuren ? Oligonucleotide aus 2',3'-Dideoxy-B-D-glucopyranosyl-Bausteine n ('Homo-DNS'): Hersellung." HELVETICA CHIMICA ACTA., Bd. 75, Nr. 5, 13. August 1992, Seiten 1416-1477, XP002096856 BASEL CH
- M.BOLLI ET AL.: "131. Pyranosyl-RNA : Further Observations on Replication." HELVETICA CHIMICA ACTA., Bd. 80, Nr. 6, 22. September 1997, Seiten 1901-1951, XP002096832 BASEL CH

## Beschreibung

Die vorliegende Erfindung betrifft ein Pentopyranosyl-Nucleosid der Formel (I) oder der Formel (II) 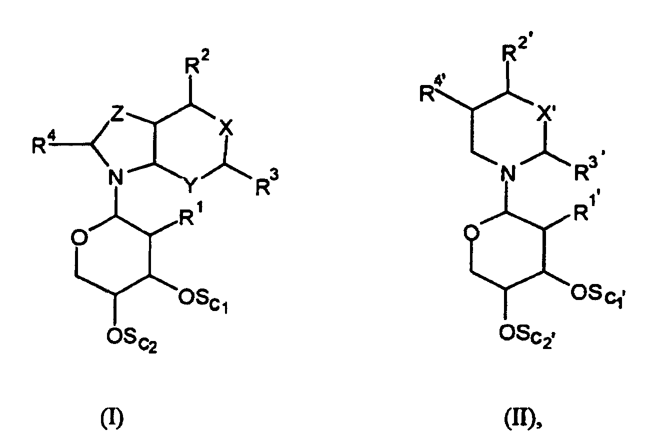 deren Herstellung und Verwendung zur Herstellung eines Therapeutikums und/oder Diagnostikums.

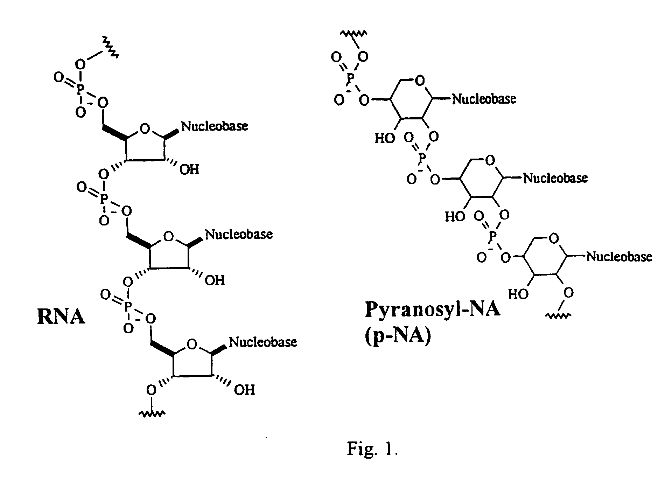

Pyranosyl-Nucleinsäuren (p-NA's) sind im allgemeinen zur natürlichen RNA isomere Strukturtypen, bei denen die Pentose-Einheiten in der Pyranoseform vorliegen und durch Phosphodiestergruppen zwischen den Positionen C-2' und C-4' repetitiv verknüpft sind (Fig. 1). Unter "Nucleobase" werden dabei die kanonischen Nucleobasen A, T, U, C, G, aber auch die Paare Isoguanin/Isocytosin und 2,6-Diaminopurin/Xanthin und im Sinne der vorliegenden Erfindung auch andere Purine und Pyrimidine verstanden. p-NA's, und zwar die von der Ribose abgeleitete p-RNA's, wurden zum erstenmal von Eschenmoser et al. beschrieben (siehe Pitsch, S. et al. Helv. Chim. Acta 1993, 76, 2161; Pitsch, S. et al. Helv. Chim Acta 1995, 78, 1621; Angew. Chem. 1996, 108, 1619-1623, Schlönvogt, I. et al. Helv. Chim Acta 1996, 79, 2316-2345). Sie bilden ausschließlich sogenannte Watson-Crickgepaarte, d. h. Purin-Pyrimidin- und Purin-Purin-gepaarte, antiparallele, reversibel "schmelzende", quasi-lineare und stabile Duplices. Homochirale p-RNA-Stränge entgegengesetzten Chiralitätssinns paaren ebenfalls kontrollierbar und sind in der gebildeten Duplex streng nicht-helical. Diese für den Aufbau supramolekularer Einheiten wertvolle Spezifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich letzlich auf die Teilnahme eines 2',4'-cisdisubstituierten Ribopyranoserings am Aufbau des Rückgrates zurüctduhren. Diese wesentlich besseren Paarungseigenschaften machen p-NA's gegenüber DNA und RNA für die Anwendung des Aufbaus supramolekularer Einheiten zu bevorzugten Paarungssystemen. Sie bilden ein zu natürlichen Nucleinsäuren orthogonales Paarungsystem, d. h. sie paaren nicht mit in der natürlich Form vorkommenden DNA's und RNA's, was im besonderen im diagnostischen Bereich von Bedeutung ist.

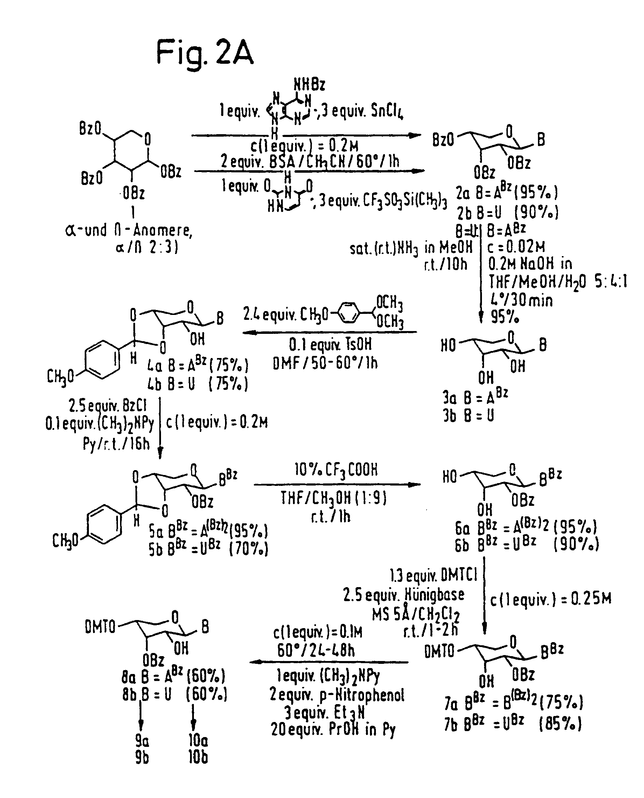

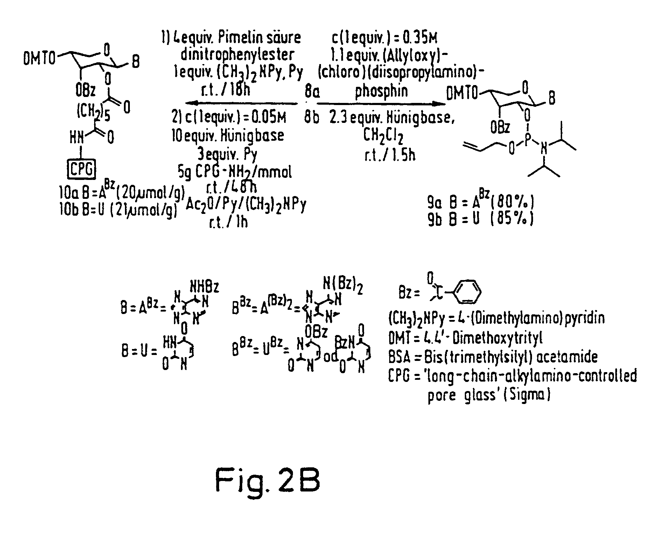

Eschenmoser et al. (1993, supra) hat zum ersten Mal eine p-RNA, wie in Fig. 2 dargestellt und nachstehend erläutert, hergestellt.

Hierbei wurde eine geeignete geschützte Nucleobase mit dem Anomerengemisch der Tetrabenzoyl-Ribopyranose durch Einwirken von Bis(trimethylsilyl)acetamid und einer Lewis-Säure wie z. B. Trimethylsilyl-trifluormethansulfonat zur Reaktion gebracht (analog H. Vorbrüggen, K. Krolikiewicz, B. Bennua, Chem. Ber. 1981, 114, 1234.). Unter Baseneinwirkung (NaOH in THF/Methanol/Wasser im Falle der Purine; gesättigter Ammoniak in MeOH im Falle der Pyrimidine) wurden die Acylschutzgruppen vom Zucker abgespalten, und das Produkt unter saurer Katalyse mit p-Anisaldehyddimethylacetal in 3',4'-Position geschützt. Das Diastereomerengemisch wurde in 2'-Stellung acyliert, das 3',4'methoxybenzylidengeschützte 2'-Benzoat durch saure Behandlung, z. B. mit Trifluoressigsäure in Methanol deacetalisiert, und mit Dimethoxytritylchlorid umgesetzt. Die 2'→3'-Wanderung des Benzoats wurde durch Behandlung mit p-Nitrophenol/4-(Dimethylamino)pyridin/Triethylamin/Pyridin/n-Propanol eingeleitet. Fast alle Reaktionen wurden durch Säulenchromatographie aufgearbeitet. Der so synthetisierte Schlüsselbaustein, das 4'-DMT-3'-benzoyl-1'-Nucleobasen-Derivat der Ribopyranose, wurde dann zum Teil phosphityliert bzw. über einen Linker an eine feste Phase gebunden.

Bei der anschließenden automatisierten Oligonucleotidsynthese wurde die trägergebundene Komponente in 4'-Position wiederholt sauer entschützt, ein Phosphoramidit unter Einwirkung eines Kupplungsreagenz, z. B. ein Tetrazolderivat, angekuppelt, noch freie 4'-Sauerstoffatome acetyliert und das Phosphoratom oxidiert, um so das oligomere Produkt.zu erhalten. Anschließend wurden die restlichen Schutzgruppen abgespalten, das Produkt über HPLC gereinigt und entsalzt.

Das beschriebene Verfahren von Eschenmoser et al. (1993, supra) zeigt jedoch folgende Nachteile:
1. Der Einsatz nicht anomerenreiner Tetrabenzoyl-pentopyranosen (H. G. Fletcher, J. Am. Chem. Soc. 1955, 77, 5337) zur Nucleosidierungsreaktion mit Nucleobasen verringert durch die Notwendigkeit rigoros geschnittener Chromatographien in den nachfolgenden Arbeitsschritten die Ausbeuten des Endproduktes.
2. Die Synthese ist mit fünf Reaktionsstufen, ausgehend von Ribopyranosen, die in 1'-Stellung eine Nucleobase aufweisen, bis zum geschützten 3'-Benzoat, sehr langwierig und eine Durchführung im industriellen Maßstab ist kaum möglich. Zusätzlich zu dem hohen Zeitaufwand sind die erhaltenen Ausbeuten an Monomerbausteinen gering: 29 % im Falle des Purinbausteins Adenin, 24 % im Falle des Pyrimidinbausteins Uracil.
3. Bei der Synthese der Oligonucleotide wird 5-(4-Nitrophenyl)-1H-tetrazol als Kupplungsreagenz in der automatisierten p-RNA-Synthese eingesetzt. Die Konzentration dieses Reagenz in der Lösung von Tetrazol in Acetonitril ist dabei so hoch, daß regelmäßig das 5-(4-Nitrophenyl)-1H-tetrazols in den dünnen Schläuchen des Synthesizers auskristallisiert und die Synthese somit zu einem vorzeitigen Ende kommt. Zudem wurde beobachtet, daß die Oligomeren mit 5-(4-Nitrophenyl)-1H-tetrazol verunreinigt waren.
4. Die beschriebene Aufarbeitung von p-RNA-Oligonucleotiden, im speziellen die Abspaltung der basenlabilen Schutzgruppen mit Hydrazinlösung, gelingt bei hohem Thymidin-Anteil im Oligomeren nicht immer.

Neben den erwähnten Veröffentlichungen von Eschenmoser et al. sind spezielle Verfahren zur Herstellung bestimmter Pyranosylverbindungen bekannt. Zu erwähnen sind dazu Doboszewski, B et al., Tetrahedron 1995, Vol. 51, No. 45, 12319-12336, worin die Herstellung von 3'-Deoxy-3'-C-Hydroxymethyl-aldopentopyranosyl-Nucleosiden beschrieben ist, Kelley, J.A., et al., J. Med. Chem., 1986, Vol. 29, No. 11, 2351-2358, worin ein Furanose-Pyranose Isomerisationsverfahren beschrieben ist und Melguizo, M. et al., J. Org. Chem., 1992, Vol. 57, Nr. 2, 559-565, worin die Synthese von 8-Aminopurin-Nucleosidanaloga beschrieben ist.

Ausgehend vom Stand der Technik war es Aufgabe der vorliegenden Erfindung Pentopyranosyl-Nucleosid-Biomolekül-Konjugate und ein Verfahren zu deren Herstellung bereitzustellen.

Die Aufgabe wird durch Konjugate enthaltend ein Pentopyranosyl-Nucleosid oder eine Pentopyranosyl-Nukleinsäure und ein Biomolekül ausgewählt aus der Gruppe der Peptide, Proteine oder Nucleinsäuren gemäß dem Anspruch 1 gelöst. Ein bevorzugter Gegenstand der vorliegenden Erfindung ist daher ein Konjugat enthaltend ein Pentopyranosyl-Nucleosid der Formel (I), 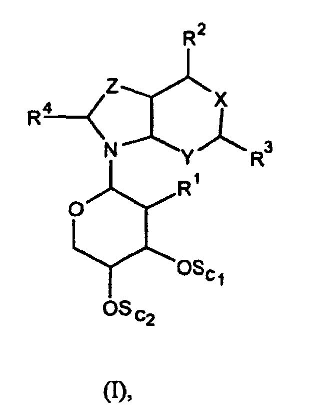 wori n
R¹ gleich H, OH, Hal mit Hal gleich Br oder Cl, oder ein Rest ausgewählt aus 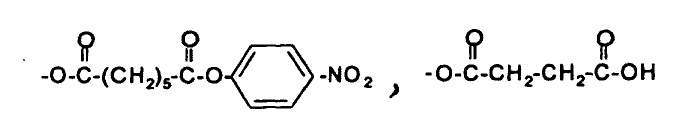 oder - O-P[N(i-Pr)₂] (OCH₂CH₂CN) mit i-Pr gleich Isopropyl, oder OS_{c3} mit S_{c3} gleich eine Schutzgruppe, vorzugsweise eine basen- oder metallkatalysiert abspaltbare Schutzgruppe, insbesondere eine Acylgruppe, besonders bevorzugt eine Benzoylgruppe, R², R³ und R⁴ unabhängig voneinander, gleich oder verschieden, jeweils H, Hal mit Hal gleich Br oder Cl, NR5R6, OR⁷, SR⁸, =O, CₙH₂ₙ₊₁ mit n eine ganze Zahl von 1-12, vorzugsweise 1-8, insbesondere 1-4, eine β-eliminierbare Gruppe, vorzugsweise eine Gruppe der Formel - OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR¹⁰R¹¹ mit R¹⁰R¹¹ gleich H, CₙH₂ₙ₊₁ oder R¹⁰R¹¹ verbunden über einen Rest der Formel 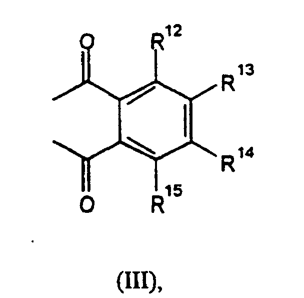 worin R¹², R¹³, R¹⁴ und R¹⁵ unabhängig voneinander, gleich oder verschieden, jeweils H, OR⁷, wobei R⁷ die oben genannte Bedeutung hat, oder CₙH₂ₙ₊₁, oder CₙH₂ₙ₋₁, wobei n die oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)- oder - N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet, und
S_{c1} und S_{c2} unabhängig voneinander, gleich oder verschieden, jeweils H oder eine Schutzgruppe ausgewählt aus einer Acyl-, Trityl- oder Allyloxycarbonylgruppe, vorzugsweise eine Benzoyl- oder 4, 4'-Dimethoxytrityl-(DMT-)gruppe,
oder der Formel (II) 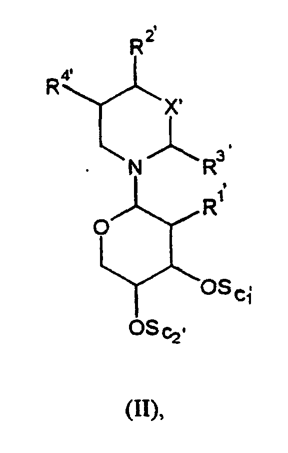 worin R^{1'} gleich H, OH, Hal mit Hal gleich Br oder Cl oder oder ein Rest ausgewählt aus 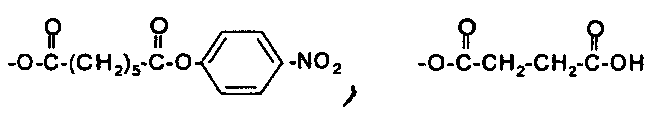 oder
- O-P[N(i-Pr)₂] (OCH₂CH₂CN) mit i-Pr gleich Isopropyl, oder OS_{c3'} mit S_{c3'} gleich eine Schutzgruppe, vorzugsweise eine basen- oder metallkatalysiert abspaltbare Schutzgruppe, insbesondere eine Acylgruppe, besonders bevorzugt eine Benzoylgruppe, R^{2'},R^{3'} und R^{4'} unabhängig voneinander, gleich oder verschieden, jeweils H, Hal mit Hal gleich Br oder Cl, =O, CₙH₂ₙ₊₁ oder OCₙH₂ₙ₋₁, eine β-eliminierbare Gruppe, vorzugsweise eine Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR^{10'}R^{11'}, wobei R^{10'}, R^{11'}, unabhängig voneinander die oben genannte Bedeutung von R¹⁰ bzw. R¹¹ hat, und
X', jeweils =N-, =C(R^{16'})- oder -N(R^{17'})- bedeutet, wobei R^{16'} und R^{17'} unabhängig voneinander die oben genannte Bedeutung von R¹⁶ bzw. R¹⁷ haben, und S_{c1'} bzw. S_{c2'} die oben genannte Bedeutung von S_{c1} bzw. S_{c2} haben.

Der erfindungsgemäße Pentopyranosyl-Nucleosid-Teil des Konjugates ist im allgemeinen ein Ribo-, Arabino-, Lyxo- und/oder Xylo-pyranosyl-Nucleosid, vorzugsweise ein Ribopyranosyl-Nucleosid, wobei der Pentopyranosyl-Teil D-konfiguriert, aber auch L-konfiguriert sein kann.

Üblicherweise handelt es sich bei dem erfindungsgemäßen Pentopyranosyl-Nucleosid-Teil des Konjugates um ein Pentopyranosyl-purin, -2,6-diaminopurin, -6-purinthiol, -pyridin, - pyrimidin, -adenosin, -guanosin, -isoguanosin, -6-thioguanosin, -xanthin, -hypoxanthin, - thymidin, -cytosin, -isocytosin , -indol, -tryptamin, -N-phthaloyltryptamin, -uracil, -coffein, - theobromin, -theophyllin, -benzotriazol oder -acridin, insbesondere um ein Pentopyranosyl-purin, -pyrimidin, -adenosin, -guanosin, -thymidin, -cytosin, tryptamin, -N-phthalotryptamin oder -uracil.

Unter die erfindungsgemäßen Verbindungen fallen auch Konjugate enthaltend Pentopyranosyl-Nucleoside, die als Linker verwendet werden können, d. h. als Unter die erfindungsgemäßen Verbindungen fallen auch Konjugate enthaltend Pentopyranosyl-Nucleoside, die als Linker verwendet werden können, d. h. als Verbindungen mit funktionellen Gruppen, die kovalent an Biomoleküle, wie z. B. in ihrer natürlichen Form vorkommende oder modifizierte Nucleinsäuren, wie DNA, RNA aber auch p-NA's, vorzugsweise pRNA's, binden können. Dies ist überraschend, da für p-NA's noch keine Linker bekannt sind.

Beispielsweise fallen hierunter Pentopyranosyl-Nucleoside, bei denen R², R³, R⁴, R^{2'}, R^{3'} und/oder R^{4'} ein 2-PhthaGmidoethyl- oder Allyloxy-Rest bedeutet. Bevorzugt sind gemäß der vorliegenden Erfindung beispielsweise Uracil-basierende Linker, bei denen vorzugsweise die 5-Position des Uracils modifiziert wurde, z. B. N-Phthaloylaminoethyluracil, aber auch Indol-basierende Linker, vorzugsweise Tryptaminderivate, wie z. B. N-Phthaloyltryptamin.

Überraschenderweise werden durch die vorliegende Erfindung auch besser handhabbare Pentopyranosyl-N,N-Diacylnucleoside, vorzugsweise Purine, insbesonders Adenosin, Guanosin oder 6-Thioguanosin, bereitgestellt, deren Nucleobase auf einfache Weise vollkommen entschützt werden können. Daher gehören zu der Erfindung auch erfindungsgemäße Pentopyranosyl-Nucleoside, bei denen R², R³, R⁴, R^{2'}, R^{3'} und/oder R^{4'} ein Rest der Formel -N[C(O)R⁹]₂ bedeutet, insbesondere N⁶, N⁶-Dibenzoyl-9-(β-Dribopyranosyl)-adenosin.

Weiterhin ist es überraschend, daß die vorliegende Erfindung Pentopyranosyl-Nucleoside bereitstellt, die ausschließlich am 3'-Sauerstoffatom des Pentopyranosid-Teils eine Schutzgruppe, vorzugsweise eine basen- oder metallkatalysiert abspaltbare Schutzgruppe, insbesondere eine Acylgruppe, besonders bevorzugt eine Benzoylgruppe, tragen. Diese Verbindungen dienen z. B. als Ausgangsstoffe zur direkten Einführung einer weiteren Schutzgruppe, vorzugsweise einer säure- oder basenlabilen Schutzgruppe, insbesondere einer Tritylgruppe, besonders bevorzugt eine Dimethoxytritylgruppe, an das 4'-Sauerstoffatom des Pentopyranosid-Teils ohne zusätzliche, die Ausbeute verringernde Schritte, wie z. B. zusätzliche Reinigungsschritte.

Darüberhinaus stellt die vorliegende Erfindung Pentopyranosyl-Nucleoside bereit, die ausschließlich am 4'-Sauerstoffatom des Pentopyranosid-Teils eine Schutzgruppe, vorzugsweise einer säure- oder basenlabilen Schutzgruppe, insbesondere einer Tritylgruppe, besonders bevorzugt eine Dimethoxytritylgruppe, tragen. Auch diese Verbindungen dienen z. B. als Ausgangsstoffe zur direkten Einführung einer weiteren Schutzgruppe, vorzugsweise einer basen- oder metallkatalysiert abspaltbaren Schutzgruppe, insbesondere einer Acylgruppe, besonders bevorzugt einer Benzoylgruppe, z. B. an dem 2'-Sauerstoffatom des Pentopyranosid-Teils, ohne zusätzliche, die Ausbeute verringernde Schritte, wie z. B. zusätzliche Reinigungsschritte.

Im allgemeinen können die erfindungsgemäßen Pentopyranosid-Nucleoside in einer sogenannten Ein-Topf-Reaktion umgesetzt werden, was die Ausbeuten erhöht und daher besonders vorteilhaft ist.

Folgende Verbindungen stellen bevorzugte Beispiele der erfindungsgemäßen Pentopyranosyl-Nucleoside dar:
A) [2',4'-Di-O-Benzoyl)-β-ribopyranosyl]-Nucleoside, insbesondere ein [2',4'-Di-O-Benzoyl)-β-ribopyranosyl]-adenin, - guanin, -cytosin, -thymidin, -uracil, -xanthin oder - hypoxanthin, sowie ein N-Benzoyl-2',4'-di-O-benzoyl-ribopyranosyl-Nucleosid, insbesondere ein -adenin, - guanin oder -cytosin, sowie ein N-Isobutyroyl-2', 4'-di-Obenzoyl-ribopyranosyl-Nucleosid, insbesondere ein -adenin, -guanin oder -cytosin, sowie ein O⁶-(2-Cyanoethyl)-N²-isobutyroyl-2',4'-di-O-benzoyl-ribopyranosyl-Nucleosid, insbesondere ein -guanin, sowie ein O⁶ (2-(4-Nitrophenyl)ethyl)-N²-isobutyroyl-2',4'-di-Obenzoyl-ribopyranosyl-Nucleosid, insbesondere ein -guanin.
B) β-Ribopyranosyl-Nucleoside, insbesondere ein β-Ribopyranosyl-adenin, -guanin, - cytosin, -thymidin oder -uracil, -xanthin oder hypoxanthin, sowie ein N-Benzoyl-, N-Isobutyroyl-, O⁶ -(2-Cyanoethyl)- oder O⁶-(2-(4-Nitrophenyl)ethyl)-N²-isobutylroyl-β-ribopyranosyl-Nucleosid.
C) 4'-DMT-pentopyranosyl-Nucleoside, vorzugsweise ein 4'-DMT-ribopyranosyl-Nucleosid, insbesondere ein 4'-DMT-ribopyranosyl-adenin, -guanin, -cytosin, -thymidin, - uracil, -xanthin, oder -hypoxanthin, sowie ein N-Benzoyl-4'-DMT-ribopyranosyl-Nucleosid, insbesondere ein N-Benzoyl-4'-DMT-ribopyranosyl-adenin, -guanin oder -cytosin, sowie ein N-Isobutyroyl-4'-DMT-ribopyranosyl-Nucleosid, insbesondere ein N-Isobutyroyl-4'-DMT-ribopyranosyl-adenin, -guanin oder -cytosin sowie ein O⁶-(2-Cyanoethyl)-N²isobutyroyl-4'-DMT-ribopyranosyl-Nucleosid, insbesondere ein O⁶-(2-Cyanoethyl)-N²isobutyroyl-4'-DMT-ribopyranosyl-guanin, sowie ein O⁶-(2-(-4-Nitrophenyl)ethyl)-N²isobutyroyl-4'-DMT-ribopyranosyl-Nucleosid, insbesondere ein O⁶-(2-(-4-Nitrophenyl)ethyl)-N²-isobutyroyl-4'-DMT-ribopyranosyl-guanin.
D) β-Ribopyranosyl-N,N'-dibenzoyl-adenosin oder β-Ribopyranosyl-N,N'-dibenzoylguanosin.

Als Vorstufe für die Oligonucleotidsynthese eigenen sich beispielsweise 4'-DMTpentopyranosyl-Nucleoside-2'-phosphitamid/-H-phosphonat, vorzugsweise ein 4'DMTribopyranosyl-Nucleosid-2'-phosphitamid/-H-phosphonat, insbesondere ein 4'-DMTribopyranosyl-adenin-, -guanin-, cytosin-, -thymidin-, -xanthin-, oder -hypoxanthin-, oder - uracil-2'-phosphitamid/-H-phosphonat sowie ein N-Benzoyl-4'-DMT-ribopyranosyl-adenin-, -guanin- oder -cytosin-2'-phosphitamid/-H-phosphonat sowie ein N-Isobutylroyl-4'-DMTribopyranosyl-adenin-, -guanin- oder -cytosin-2'-phosphitamid/-H-phosphonat, O⁶-(2-Cyanoethyl)-4'-DMT-ribopyranosyl-guanin-, -xanthin-, -hypoxanthin-2'-phosphitamid/-Hphosphonat oder O⁶-(2-(4-Nitrophenyl)ethyl)-N²-isobutyroyl-4'-DMT-ribopyranosyl-guanin und für die Kopplung an den festen Träger beispielsweise 4'-DMT-pentopyranosyl-Nucleoside-2'-succinat, vorzugsweise ein 4'-DMT-ribopyranosyl-Nucleosid-2'-succinat, insbesondere ein 4'DMT-ribopyranosyl-adenin-, -guanin-, -cytosin-, -thymidin-, -xanthin-, - hypoxanthin- oder -uracil-2'-succinat sowie ein N-Benzoyl-4'-DMT-ribopyranosyl-adenin-, - guanin- oder -cytonsin-2'-succinat sowie ein N-Isobutyroyl-4'-DMT-ribopyranosyl-adenin-, -guanin- oder -cytosin-2'- succinat, O-(2-Cyanoethyl)-4'-DMT-ribopyranosyl-guanin-, - xanthin- oder -hypoxanthin-2'- succinat sowie ein O⁶-(2-(4-Nitrophenyl)ethyl)-N²isobutyroyl-4'-DMT-ribopyranosyl-guanin-2'-succinat.

Ein weiterer Gegenstand der vorliegenden Erfindung ist ein Verfahren zur Herstellung eines erfindungsgemäßen Pentopyranosyl-Nucleosids der Formel (I) oder (II), bei dem ausgehend von dem ungeschützten Pentopyranosid
(a) in einem ersten Schritt zuerst die 2'-, 3'- oder 4'-Position des Pentopyranosids geschützt wird, und vorzugsweise
(b) in einem zweiten Schritt die andere Position an der 2'-, 3'- oder 4'-Position.

Das erfindungsgemäße Verfahren ist nicht auf die in der zitierten Literatur beschriebenen Nucleobasen beschränkt, sondern kann überraschenderweise mit einer Vielzahl von natürlichen und synthetischen Nucleobasen erfolgreich durchgeführt werden. Zudem ist es besonders überraschend, daß das erfindungsgemäße Verfahren im Vergleich zu dem literaturbekannten Verfahren in großen Ausbeuten und mit einer Zeitersparnis von durchschnittlich 60% durchgeführt werden kann, was für die industrielle Anwendung besonders vorteilhaft ist. Zusätzlich sind mit dem erfindungsgemäßen Verfahren die bei dem in der Literatur beschriebenen Verfahren nötigen Reinigungsschritte, z. B. chromatographische Zwischenreinigungen, nicht notwendig und die Reaktionen können teilweise als sogenannnte Ein-Topf Reaktion durchgeführt werden, was die Raum/Zeit-Ausbeuten deutlich erhöht.

In einer besonderen Ausführungsform erfolgt im Falle einer 2'-geschützten Position eine Umlagerung der Schutzgruppe von der 2'-Position zur 3'-Position, die im allgemeinen in Gegenwart einer Base, insbesondere in Gegenwart von N-Ethyldiisopropylamin und/oder Triethylamin durchgeführt wird. Diese Reaktion kann gemäß der vorliegenden Erfindung besonders vorteilhaft in dem gleichen Reaktionsbehältnis als Ein-Topf-Reaktion durchgeführt werden.

In einer weiteren bevorzugten Ausführungsform ist das Pyranosyl-nucleosid durch eine säurelabile, basenlabile oder metallkatalysiert abspaltbare Schutzgruppe S_{c1}, S_{c2}, S_{c1'} oder S_{c2'} geschützt, wobei vorzugsweise die Schutzgruppen S_{c1} und S_{c1'} von den Schutzgruppen S_{c2} bzw. S_{c2'} verschieden sind.

Im allgemeinen handelt es sich bei den genannten Schutzgruppen um eine Acylgruppe, vorzugsweise um eine Acetyl-, Benzoyl-, Nitrobenzoyl- und/oder Methoxybenzoyl-Gruppe, um Tritylgruppen, vorzugsweise um eine 4, 4'-Dimethoxytrityl-(DMT)-Gruppe oder um eine β-eliminierbare Gruppe, vorzugsweise eine Gruppe der Formel -OCH₂CH₂R¹⁸ mit R¹⁸ gleich ein Cyano- oder p-Nitrophenylrest oder eine Fluorenylmethyloxycarbonyl-(Fmoc)Gruppe.

Besonders bevorzugt ist es, wenn die 2'- oder 3'-Position durch eine basenlabile oder metallkatalysiert abspaltbare Schutzgruppe, vorzugsweise durch eine Acylgruppe, insbesondere durch eine Acetyl-, Benzoyl-, Nitrobenzoyl- und/oder Methoxybenzoylgruppe, geschützt wird und/oder die 4'-Position durch eine säure- oder basenlabile Schutzgruppe, vorzugsweise durch eine Trityl- und/oder Fmoc-Gruppe, insbesondere durch eine DMT-Gruppe geschützt wird.

Das erfindungsgemäße Verfahren kommt folglich im Gegensatz zu dem literaturbekannten Verfahren ohne acetalische Schutzgruppen, wie Acetale oder Ketale, aus, was zusätzliche chromatographische Zwischenreinigungen vermeidet und folglich die Reaktionen als Ein-Topf-Reaktionen mit überraschend hohen Raum/Zeit-Ausbeuten durchführen läßt.

Die Einführung der genannten Schutzgruppen erfolgt vorzugsweise bei tiefen Temperaturen, da diese hierdurch überraschenderweise selektiv eingeführt werden können.

So erfolgt beispielsweise die Einführung einer Benzoylgruppe durch Umsetzen mit Benzoylchlorid in Pyridin bzw. in einem Pyridin/Methylenchlorid-Gemisch bei tiefen Temperaturen. Die Einführung einer DMT-Gruppe kann beispielsweise durch Umsetzen mit DMTCl in Anwesenheit einer Base, z. B. von N-Ethyldiisopropylamin (Hünig-Base), und z. B. von Pyridin, Methylenchlorid oder einem Pyridin/Methylenchlorid-Gemisch bei Raumtemperatur erfolgen.

Es ist auch vorteilhaft, wenn nach der Acylierung und/oder nach der gegebenenfalls erfolgten Umlagerung von der 2'- zu der 3'-Position die Reaktionsprodukte chromatographisch gereinigt werden. Eine Reinigung nach der Tritylierung ist gemäß dem erfindungsgemäßem Verfahren nicht notwendig, was besonders vorteilhaft ist.

Das Endprodukt kann, falls notwendig, noch durch Kristallisation weiter gereinigt werden.

Ein anderer Gegenstand der vorliegenden Erfindung ist ein Verfahren zur Herstellung eines Ribopyranosyl-nucleosids, bei dem
(a) eine geschützte Nucleobase mit einer geschützten Ribopyranose umgesetzt wird,
(b) die Schutzgruppen von dem Ribopyranosyl-Teil des Produktes aus Schritt (a) abgespalten werden, und
(c) das Produkt aus Schritt (b) gemäß dem oben näher beschriebenen Verfahren umgesetzt wird.

Hierbei ist es zur Vermeidung weiterer zeit- und materialaufwendiger Chromatographien vorteilhaft, nur anomerenreine geschützte Pentopyranosen, wie z. B. Tetrabenzoyl-pentopyranosen, vorzugsweise β-Tetrabenzoyl-ribopyranosen (R. Jeanloz, J. Am. Chem. Soc. 1948, 70, 4052), einzusetzen.

In einer weiteren Ausführungsform wird ein Linker gemäß Formel (II), worin R^{4'} (CₙH₂ₙ)NR^{10'}R^{11'} bedeutet und R^{10'}R^{11'} über einen Rest der Formel (III) mit der bereits bezeichneten Bedeutung verbunden ist, durch folgendes Verfahren auf vorteilhafte Weise hergestellt:
(a) eine Verbindung der Formel (II) mit R^{4'} gleich (CₙH₂ₙ)OS_{c3} oder (CₙH₂ₙ)Hal, worin n die oben genannte Bedeutung hat, S_{c3} eine Schutzgruppe, vorzugsweise eine Mesylat-Gruppe, und Hal Chlor oder Brom bedeutet, wird mit einem Azid, vorzugsweise in DMF, umgesetzt, anschließend wird
(b) das Reaktionprodukt aus (a), vorzugsweise mit Triphenylphosphin z. B. in Pyridin reduziert, dann
(c) das Reaktionsprodukt aus (b) mit einem entsprechenden Phthalimid, z. B. N-Ethoxycarbonylphthalimid, umgesetzt, und
(d) das Reaktionsprodukt aus (c) mit einer entsprechenden geschützten Pyranose, z. B. Ribosetetrabenzoat, umgesetzt, und schließlich
(e) werden die Schutzgruppen, z. B. mit Methylat, abgespalten, und
(f) die weiteren Schritte, wie oben bereits beschrieben, durchgeführt.

Daneben weisen Indolderivate als Linker den Vorzug der Fluoreszenzfähigkeit auf und sind daher für Nanotechnologie-Anwendungen, bei denen es ggf. um den Nachweis kleinster Substanzmengen geht, besonders bevorzugt. So wurden Indol-1-riboside bei N. N. Suvorov et al., Biol. Aktivn. Soedin., Akad. Nauk SSSR 1965, 60 und Tetrahedron 1967, 23, 4653 bereits beschrieben. Allerdings gibt es kein analoges Verfahren, 3-substituierte Derivate herzustellen.. Im allgemeinen erfolgt ihre Herstellung über die Bildung eines Aminals der ungeschützten Zuckerkomponente und einem Indolin, welches dann durch Oxidation in das Indol-1-ribosid übergeführt wird. Beschrieben wurden z. B. Indol-1-glucoside und -1-arabinoside (Y. V. Dobriynin et al, Khim.-Farm. Zh. 1978, 12, 33), deren 3-substituierte Derivate meist über Vielsmeier-Reaktion hergestellt wurden. Dieser Weg der Einführung von Aminoethyl-Einheiten in 3-Position des Indols ist für eine industrielle Anwendung jedoch zu aufwendig.

In einer weiteren besonderen Ausführungsform wird daher ein Linker gemäß Formel (I), worin X und Y unabhängig voneinander, gleich oder verschieden, jeweils =C(R¹⁶) mit R¹⁶ gleich H oder CₙH₂ₙ und Z =C(R¹⁶)- mit R¹⁶ gleich (CₙH₂ₙ)NR¹⁰R¹¹ durch folgendes Verfahren auf vorteilhafte Weise hergestellt:
(a) das entsprechende Indolin, z. B. N-Phthaioyltryptamin, wird mit einer Pyranose, z. B. D-Ribose, zum Nucleosidtriol umgesetzt, dann werden
(b) die Hydroxylgruppen des Pyranosyl-Teils des Produktes aus (a) vorzugsweise mit Acylgruppen, z. B. mittels Essigsäureanhydrid, geschützt, anschließend wird
(c) das Produkt aus (b), z. B. durch 2,3-Dichlor-5,6-dicyanoparachinon, oxidiert, und
(d) die Hydroxyl-Schutzgruppen des Pyranosyl-Teils des Produktes aus (c) werden z. B. mittels Methylat abgespalten und anschließend werden
(e) die weiteren Schritte, wie oben bereits beschrieben, durchgeführt.

Dieses Verfahren läßt sich jedoch nicht nur bei Ribopyranosen anwenden, sondern auch bei Ribofuranosen und 2'-Deoxyribofuranosen bzw. 2'-Deoxyribopyranosen, was besonders vorteilhaft ist. Als Nucleosidierungspartner der Zucker wird vorzugsweise Tryptamin, insbesondere N-Acylderivate des Tryptamins, vor allem N-Phthaloyltryptamin verwendet.

In einer weiteren Ausführungsform werden die 4'- geschützten, vorzugsweise, die 3', 4'geschützten Pentopyranosyl-Nucleoside in einem weiteren Schritt phosphityliert oder an eine feste Phase gebunden.

Die Phosphitylierung erfolgt beispielsweise durch Phosphorigsäuremonoallylesterdiisopropyl-amidchlorid in Anwesenheit einer Base, z. B. N-Ethyldiisopropylamin oder durch Phosphortrichlorid und Imidazol bzw. Tetrazol und nachfolgender Hydrolyse unter Basenzusatz. Im ersten Fall ist das Produkt ein Phosphoramidit und im zweiten Fall ein H-Phosphonat. Die Bindung eines geschützten erfindungsgemäßen Pentopyranosyl-Nucleosids an eine feste Phase, z. B. "long-chain-alkylamino-controlled pore glass" (CPG, Sigma Chemie, München) kann beispielsweise wie bei Eschenmoser et al. (1993) beschrieben erfolgen.

Die erhaltenen Verbindungen dienen z. B. für die Herstellung von Pentopyranosyl-Nucleinsäuren.

Ein weiterer Gegenstand der vorliegenden Erfindung ist daher ein Verfahren zur Herstellung einer Pentopyranosyl-Nucleinsäure, mit folgenden Schritten:
(a) in einem ersten Schritt wird ein geschütztes Pentopyranosyl-Nucleosid, wie oben bereits beschrieben, an eine feste Phase gebunden wird und
(b) in einem zweiten Schritt wird das gemäß Schritt (a) an eine feste Phase gebundene 3'-, 4'-geschützte Pentopyranosylnukleosid um ein phosphityliertes 3'-, 4'-geschütztes Pentopyranosyl-Nucleosid verlängert und anschließend z. B. durch eine wäßrige Jodlösung oxidiert wird, und
(c) Schritt (b) solange mit gleichen oder unterschiedlichen phosphitylierten 3'-, 4'geschützten Pentopyranosyl-Nucleosiden wiederholt, bis die gewünschte Pentopyranosyl-Nucleinsäure vorliegt.

Als Kupplungsreagenz bei Einsatz von Phosphoramiditen eignen sich saure Aktivatoren wie Pyridinium-Hydrochlorid besonders Benzimidazoliumtriflat, vorzugsweise nach Umkristallisieren in Acetonitril und nach Lösen in Acetonitril, da im Gegensatz zu 5-(4-Nitrophenyl)-1H-tetrazol als Kupplungsreagenz keine Verstopfung der Kupplungsreagenz-Leitungen und eine Verunreinigung des Produktes erfolgt.

Als Kupplungsreagenz beim Einsatz von H-Phosphonaten eignen sich besonders Arylsulfonylchloride, Diphenylchlorophosphat, Pivaloylchlorid oder Adamantoylchlorid.

Weiterhin ist es vorteilhaft durch Zusatz von einem Salz, wie Natriumchlorid, zur schutzgruppenabspaltenden Hydrazinolyse von Oligonukleotiden, insbesondere von p-NA's, vorzugsweise von p-RNA's, Pyrimidinbasen, vor allem Uracil und Thymin, vor einer Ringöffnung zu schützen, die das Oligonukleotid zerstören würde. Allyloxygruppen können vorzugsweise durch Palladium [Pd(0)]-Komplexe z. B. vor der Hydrazinolyse abgespalten werden.

In einer weiteren besonderen Ausführungsform können in Schritt (a) und/oder Schritt (b) auch Pentofuranosyl-nucleoside, z. B. das in ihrer natürlichen Form vorkommende Adenosin, Guanosin, Cytidin, Thymidin und/oder Uracil, eingebaut werden, was z. B. zu einer gemischten p-NA-DNA bzw. p-NA-RNA führt.

In einer anderen besonderen Ausführungsform kann in einem weiteren Schritt ein Allyloxy-Linker der Formel

S_{c4}NH(CₙH₂ₙ)CH(OPS_{c5}S_{c6})CₙH₂ₙOS_{c7} (IV),

worin S_{c4} und S_{c7} unabhängig voneinander, gleich oder verschieden, jeweils eine Schutzgruppe insbesondere ausgewählt aus Fmoc und/oder DMT,
S_{c5} und S_{c6} unabhängig voneinander, gleich oder verschieden, jeweils eine Allyloxyund/oder Diisopropylamino-Gruppe bedeuten, eingebaut werden. n hat die bereits oben genannte Bedeutung.

Ein besonders bevorzugter Allyloxy-Linker ist (2-(S)-N-Fmoc-O¹-DMT-O²allyloxydiisopropylaminophosphinyl-6-amino-1,2-hexandiol).

Ausgehend von z. B. Lysin können somit in wenigen Reaktionsschritten amino-terminale Linker aufgebaut werden, die sowohl eine aktivierbare Phosphorverbindung als auch eine säurelabile Schutzgruppe, wie DMT, tragen und daher leicht in der automatisierbaren Oligonucleotidsynthese verwendet werden können (siehe z. B. P. S. Nelson et al., Nucleic Acid Res. 1989, 17, 7179; L. J. Amold et al., WO 8902439). Das Repertoire wurde in der vorliegenden Erfindung durch einen Lysin-basierender Linker erweitert, bei dem anstelle der sonst üblichen Cyanoethyl-Gruppe am Phosphoratom eine Allyloxy-Gruppe eingebracht wurde, und welcher daher in vorteilhafter Weise in der Noyori-Oligonucleotid-Methode eingesetzt werden kann (R. Noyori, J. Am. Chem. Soc. 1990, 112, 1691-6).

Ein weiterer Gegenstand der vorliegenden Erfindung ist daher auch eine Pentopyranosyl-Nucleinsäure, die mindestens ein erfindungsgemäßes Pentopyranosyl-Nucleosid und gegebenenfalls mindestens einen Allyloxy-Linker enthält.

p-NA's und insbesondere die p-RNA's bilden untereinander stabile Duplices und paaren im allgemeinen nicht mit den in ihrer natürlichen Form vorkommenden DNA's und RNA's. Diese Eigenschaft macht p-NA's zu bevorzugten Paarungssystemen.

Solche Paarungssysteme sind supramolekulare Systeme nicht kovalenter Wechselwirkung, die sich durch Selektivität, Stabilität und Reversiblität auszeichnen, und deren Eigenschaften bevorzugt thermodynamisch, d. h. durch Temperatur, pH-Wert und Konzentration beeinflußt werden. Solche Paarungssysteme können z. B. aufgrund ihrer selektiven Eigenschaften auch als "molekularer Klebstoff" für die Zusammenführung von unterschiedlichen Metallclustern zu Cluster-Verbänden mit potentiell neuen Eigenschaften verwendet werden [siehe z. B. R. L. Letsinger, et al., Nature 1996, 382, 607-9; P. G. Schultz et al., Nature 1996, 382, 609-11]. Folglich eignen sich die p-NA's auch für die Anwendung im Bereich der Nanotechnologie, beispielsweise zur Herstellung neuer Materialien, Diagnostika und Therapeutika sowie mikroelektronischer, photonischer bzw. optoelektronischer Bauteile und für das kontrollierte Zusammenführen molekularer Species zu supramolekularen Einheiten, wie z. B. für den (kombinatorischen) Aufbau von Proteinassemblies [siehe z. B. A. Lombardi, J. W. Bryson, W. F. DeGrado, Biomoleküls (Pept. Sci.) 1997, 40, 495-504], da p-NA's Paarungssysteme bilden, die stark und thermodynamisch kontrollierbar sind. Eine weitere Anwendung ergibt sich daher gerade im diagnostischen und drug discovery-Bereich durch die Möglichkeit, funktionelle, bevorzugt biologische Einheiten wie Proteine oder DNA/RNA-Abschnitte, mit einem p-NA-Code zu versehen, der nicht mit den natürlichen Nucleinsäuren interferiert (siehe z. B. WO93/20242).

Ein anderer Gegenstand der Erfindung ist daher die Verwendung eines erfindungsgemäßen Pentopyranosyl-Nucleosid der Formel (I) oder (II) oder einer erfindungsgemäßen Pentopyranosyl-Nucleinsäure zur Herstellung eines Arzneimittels, wie z.B. eines Therapeutikums, eines Diagnostikums und/oder eines elektronischen Bauteils.

Ein Biomolekül, z. B. DNA oder RNA, kann zum nicht-kovalenten Verbinden (Linken) mit einem anderen Biomolekül, z. B. DNA oder RNA, verwendet werden, wenn beide Biomoleküle Abschnitte enthalten, die aufgrund komplementärer Sequenzen von Nucleobasen durch Ausbildung von Wasserstoffbrücken aneinander binden können. Derartige Biomoleküle finden z. B. in analytischen Systemen zur Signalamplifizierung Verwendung, wo ein in seiner Sequenz zu analysierendes DNA-Molekül über einen solchen nicht-kovalenten DNA-Linker zum einen an einen festen Träger immobilisiert, und zum anderen an ein signalverstärkendes branchedDNA-Molekül (bDNA) gebunden werden soll (siehe z.B. S. Urdea, Bio/Technol. 1994, 12, 926 oder US-Patent Nr. 5,624,802). Ein wesentlicher Nachteil der zuletzt beschriebenen Systeme ist, daß sie den Verfahren zur Nucleinsäure-Diagnostik durch Polymerase-Chain-Reaction (PCR) (K. Mullis, Methods Enrymol. 1987, 155, 335) hinsichtlich der Empfindlichkeit bis jetzt unterlegen sind. Das ist u. a. darauf zurückzuführen, daß die nicht-kovalente Bindung vom festen Träger an das zu analysierende DNA-Molekül ebenso wie die nicht-kovalente Bindung des zu analysierenden DNA-Moleküls nicht immer spezifisch erfolgt, wodurch es zu einer Vermischung der Funktionen "Sequenzerkennung" und "nicht-kovalente Bindung" kommt. Die Verwendung von p-NA's als orthogonales Paarungssystem, welches nicht in das DNA- bzw. RNA-Paarungsgeschehen eingreift, löst dieses Problem auf vorteilhafte Weise, wodurch die Empfindlichkeit der beschriebenen analytischen Verfahren deutlich erhöht werden kann.

Ein weiterer Gegenstand der vorliegenden Erfindung ist daher die Verwendung eines erfindungsgemäßen Pentopyranosyl-Nucleosids der Formel (I) oder (II) oder einer erfindungsgemäßen Pentopyranosyl-Nucleinsäure zur Herstellung eines Konjugates enthaltend ein erfindungsgemäßes Pentopyranosyl-Nucleosid der Formel (I) oder (II) oder eine erfindungsgemäße Pentopyranosyl-Nucleinsäure und ein Biomolekül.

Konjugate sind im Sinne der vorliegenden Erfindung kovalent gebundene Hybride aus p-NA's und anderen Biomolekülen, vorzugsweise ein Peptid, Protein oder eine Nucleinsäure, beispielsweise ein Antikörper oder ein funktioneller Teil davon oder eine in ihrer natürlichen Form vorkommende DNA und/oder RNA. Funktionelle Teile von Antikörper sind beispielsweise Fv-Fragmente (Skerra & Plückthun (1988) Science 240, 1038), einzelkettige Fv-Fragmente (scFv; Bird et al. (1988), Science 242, 423; Huston et al. (1988) Proc. Natl. Acad. Sci. U.S.A., 85, 5879) oder Fab-Fragmente (Better et al. (1988) Science 240, 1041).

Unter Biomolekül versteht man im Sinne der vorliegenden Erfindung eine natürlich vorkommende Substanz oder eine von einer natürlich vorkommenden Substanz abgeleitete Substanz.

In einer bevorzugten Ausführungsform handelt es sich dabei um p-RNA/DNA- bzw p-RNA/RNA-Konjugate.

Konjugate werden dann vorzugsweise verwendet, wenn die Funktionen "Sequenzerkennung" und .,nicht-kovalente Bindung" in einem Molekül realisiert werden müssen, da die erfindungsgemäßen Konjugate zwei zueinander orthogonale Paarungssysteme enthalten.

Für die Herstellung von Konjugaten sind sowohl sequentielle als auch konvergente Verfahren geeignet.

In einem sequentiellen Verfahren wird z. B. nach erfolgter automatisierter Synthese eines p-RNA-Oligomeren direkt am gleichen Synthesizer - nach Umstellung der Reagenzien und des Kupplungsprotokolls - z. B. ein DNA-Oligonukleotid weitersynthetisiert. Dieser Vorgang läßt sich auch in umgekehrter Reihenfolge durchführen.

In einem konvergenten Verfahren werden z. B. p-RNA-Oligomere mit Aminoterminalen-Linkern und z. B. DNA-Oligomere mit z. B. Thiol-Linkern in getrennten Vorgängen synthetisiert. Anschließend erfolgt vorzugsweise eine Jodacetylierung des p-RNA-Oligomeren und die Kupplung der beiden Einheiten nach literaturbekannten Protokollen (T. Zhu et al., Bioconjug. Chem. 1994, 5, 312).

Konvergente Verfahren erweisen sich aufgrund ihrer Flexibilität als besonders bevorzugt.

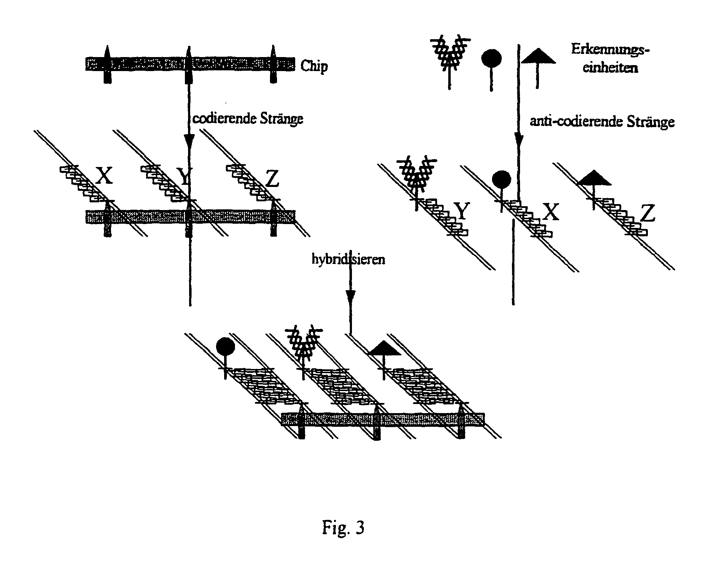

Unter dem Begriff Konjugat im Sinne der vorliegenden Erfindung sind auch sogenannte Arrays zu verstehen. Arrays sind Anordnungen von immobilisierten Erkennungsspecies, die speziell in der Analytik und Diagnostik eine wichtige Rolle bei der simultanen Bestimmung von Analyten spielen. Beispiele sind Peptide-Arrays (Fodor et al., Nature 1993, 364, 555) und Nucleinsäure-Arrays (Southern et al. Genomics 1992, 13, 1008; Heller, US-Patent Nr. 5,632,957). Eine höhere Flexibilität dieser Arrays kann dadurch erreicht werden, daß die Erkennungsspecies an codierende Oligonucleotide gebunden werden und die zugehörigen, komplementären Stränge an bestimmte Positionen auf einem festen Träger. Durch Aufbringen der codierten Erkennungsspecies auf den "anti-codierten" festen Träger und Einstellung von Hybridisierungsbedingungen werden die Erkennungsspecies an den gewünschten Positionen nicht-kovalent gebunden. Dadurch können verschiedene Typen von Erkennungsspecies, wie z. B. DNA-Abschnitte, Antikörper, nur durch Anwendung von Hybridisierungsbedingungen gleichzeitig auf einem festen Träger angeordnet werden (siehe Fig. 3.). Voraussetzung hierzu sind aber äußerst starke, selektive - um die codierenden Abschnitte möglichst kurz zu halten - und mit natürlicher Nucleinsäure nicht interferierender Codons und Anticodons notwendig. p-NA's, vorzugsweise p-RNA's eignen sich hierzu in besonders vorteilhafter Weise.

Unter dem Begriff "Träger" versteht man im Sinne der vorliegenden Erfindung Material, insbesondere Chipmaterial, das in fester oder auch gelartiger Form vorliegt. Als Trägermaterialien eignen sich beispielsweise Keramik, Metall, insbesondere Edelmetall, Gläser, Kunststoffe, kristalline Materialien bzw. dünne Schichten des Trägers, insbesondere der genannten Materialien bzw. dünne Schichten des Trägers, insbesondere der genannten Materialien, oder (bio)molekulare Filamente wie Cellulose, Gerüstproteine.

Die vorliegende Erfindung betrifft daher auch die Verwendung der erfindungsgemäßen Pentopyranosyl-Nucieinsäuren, vorzugsweise Ribopyranosyl-Nucleinsäuren zur Codierung von Erkennungsspecies, bevorzugt natürliche DNA- oder RNA-Stränge oder Proteine, insbesondere Antikörper oder funktionelle Teile von Antikörpern. Diese können dann gemäß Fig. 3. mit den zugehörigen Codons auf einem festen Träger hybridisiert werden. Damit kann auf einem festen Träger, der in Form eines Arrays mit Codons ausgestattet ist, nur durch Einstellung von Hybridisierungsbedingungen mit immer neuen Kombinationen von Erkennungsspecies an den gewünschten Positionen immer neue, diagnostisch nützliche Arrays aufgebaut werden. Wird dann der Analyt, beispielsweise eine biologische Probe wie Serum o. ä. aufgebracht, dann werden die zu detektierenden Species in einem bestimmten Muster auf dem Array gebunden, welches dann indirekt (z. B. durch Fluoreszenzmarkierung der Erkennungsspecies) oder direkt (z. B. durch Impedanzmessung am Anknüpfungspunkt der Codons) registriert wird. Dann wird die Hybridisierung durch geeignete Bedingung aufgehoben (Termperatur, Salze, Lösungsmittel, elektrophoretische Vorgänge) so daß wieder nur der Träger mit den Codons zurückbleibt. Dieser wird dann erneut mit anderen Erkennungsspecies beladen und wird z. B. für den gleichen Analyten für die Ermittlung eines anderen Musters verwendet. Die immer neue Anordnung von Erkennungsspecies im Array-Format und die Verwendung von p-NA's als Paarungssysteme ist gegenüber anderen Systemen. siehe z. B. WO 96/13522 (s. 16, unten), besonders vorteilhaft.

Ein weiterer Gegenstand der vorliegenden Erfindung bezieht sich daher insbesondere auch auf
ein Diagnostikum enthaltend ein oben beschriebenes Pentopyranosyl-Nucleosid oder ein erfindungsgemäßes Konjugat, wie oben bereits näher beschrieben.

Die folgenden Figuren und Beispiele sollen die Erfindung näher beschreiben, ohne sie zu beschränken.

### BESCHREIBUNG DER FIGUREN

- Fig. 1: zeigt einen Ausschnitt aus der Struktur von RNA in ihrer natürlich vorkommenden Form (links) und in Form einer p-NA (rechts).
- Fig.2: zeigt schematisch die Synthese eines p-Ribo(A,U)-Oligonucleotids nach Eschenmoser et al. (1993).
- Fig. 3: zeigt schematisch eine Anordnung von immobilisierten Erkennungsstrukturen (Arrays) auf einem festen Träger.

### BEISPIELE

### Beispiel 1

### Synthese des 1-{3'-O-Benzoyl-4'-O-[(4,4'-dimethoxytriphenyl)-methyl]-β-D-ribo-pyranosyl}-thymins

Zuerst 4'-Substitution, dann 2'-Substitution, dann Wanderungsreaktion: 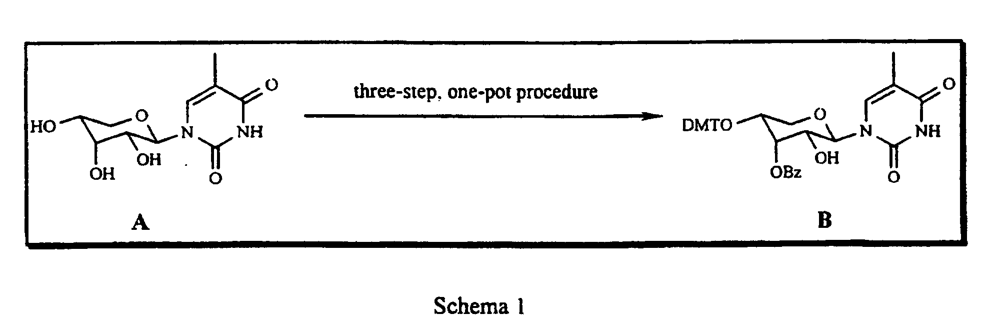 Unter Argonatmosphäre wurden 51,6 g (200 mmol) 1-(β-D-Ribo-pyranosyl)-thymin A in 620 ml wasserfreiem Pyridin gelöst, 71,4 ml (2,1 eq.) N-Ethyldiisopropylamin und 100 g Molsieb (4 Å) zugesetzt und 15 min lang mit KPG-Rührer gerührt. Man löste 92 g (272 mmol; 1,36 eq.) Dimethoxytritylchlorid (DMTCI) in 280 ml (frisch von festem NaHCO₃ destilliertem) Chloroform, und tropfte diese Lösung innerhalb von 30 min bei -6 bis -5 °C der Triollösung zu. Es wurde 1 h bei dieser Temp., dann über Nacht bei Raumtemperatur (RT.) gerührt, wieder gekühlt, und weitere 25 g (74 mmol; 0,37 eq.) DMTCI in 70 ml Chloroform zugesetzt. Man ließ auf RT. kommen und rührte 4 h nach.
Eine kleine Probe wurde entnommen, wäßrig aufgearbeitet und chromatographiert, um die analytischen Daten des 1-{4'-O-[(4,4'-dimethoxytriphenyl)-methyl]-β-D-ribo-pyranosyl}thymins zu erhalten:

^{**1**}**H-NMR** (300 MHz, CDCl3): 1,70 (bs, 2H, OH); 1,84 (d, 3H, Me); 2,90 (bs, 1 H, OH); 3,18, 3,30 (2m, 2H, H(5')), 3,62 (bs, 1H, H(3')); 3,70-3,82 (m, 8 H, 2 OMe, H(4'), H(2')); 5,75 (d, J = 9,5 Hz, 1H, H(1')), 6,85 (m, 4H, Harom); 6,96 (m, 1 H, Harom), 7,20 (m, 9H, Harom, H(6)), 8,70 (bs, 1H, H(3).)

Das Reaktionsgemisch wurde mit 2,46 g (20,5 mmol; 0,1 eq.) 4-Dimethylaminopyridin (DMAP) versetzt, auf -6 °C gekühlt und zwischen -6 und -1 °C innerhalb von 15 min 27,9 ml (0,24 mol; 1,2 eq.) Benzoylchlorid (BzCl) in 30 ml Pyridin zugetropft und 10 min nachgerührt. Zur vollständigen Umsetzung wurden dann im Abstand von 25 min noch jeweils 2,8 ml (24 mmol; 0,12 eq.) BzCl unter Kühlung zugesetzt und schließlich 20 min gerührt.

Man setzte bei RT. 460 ml wasserfreies Pyridin. 841 ml (11,2 mol: 56 eq.) n-Propanol, 44 g (0,316 mol; 1,58 eq.) p-Nitrophenol, 21,7 g (0,18 mol; 0,9 eq.) DMAP und 136 ml (0,8 mol; 4 eq.) N-Ethyldiisopropylamin zu und rührte bei 61 - 63 °C 48 h lang. Dann blieb der Ansatz bei RT. 60 h stehen. Das Reaktionsgemisch wurde noch 24 h lang auf 61 - 63 °C erwärmt, auf RT. abgekühlt und am Rotavapor eingeengt. Der Rückstand wurde in 2 l Ethylacetat aufgenommen, das Molsieb abfiltriert, die org. Phase dreimal mit je 1 l Wasser extrahiert, einmal mit 1,2 l 10 %iger Citronensäure 5 min ausgerührt und die org. Phase wieder abgetrennt, einmal mit 1 l Wasser und zum Abschluß mit 1 l gesättigter NaHCO₃-Lösung extrahiert. Die org. Phase wurde mit Natriumsulfat getrocknet, filtriert und eingeengt (220 g Rückstand).

Man filtrierte zuerst über Kieselgel 60 (20 x 10 cm) mit Stufengradienten Heptan/Ethylacetat, 1:1 bis 0:1) zur Vorreinigung, dann wurde an Kieselgel 60 chromatographiert (30 x 10 cm; Stufengradient Dichlormethan/Ethylacetat, 1:0 bis 1:1).

Man erhielt: 40 g unpolare Fraktionen
52,9 g 1-{3'-O-Benzoyl-4'-O-[(4,4'-dimethoxytriphenyl)-methyl]-β-D-ribo-pyranosyl}-thymin B
34,5 g verunreinigtes B
3,4 g polare Fraktionen

Die verunreinigte Fraktion wurde erneut chromatographiert (KG 60, 45 x 10 cm; Dichlormethan/Ethylacetat, 3:1) und lieferte weitere 11,3 g **B.**

Gesamtausbeute: 64,2 g (97 mmol) **B,** d. s. 48 % Ausbeute. ¹H-NMR entspricht.

### Beispiel 2

### Synthese des N⁴-Benzoyl-1-{3'-O-benzoyl-4'-O-[(4,4'-dimethoxytriphenyl)-methyl]-β-Dribo-pyranosyl}-cytosins

Zuerst 2'-Substitution, dann 4'-Substitution, dann Wanderungsreaktion:

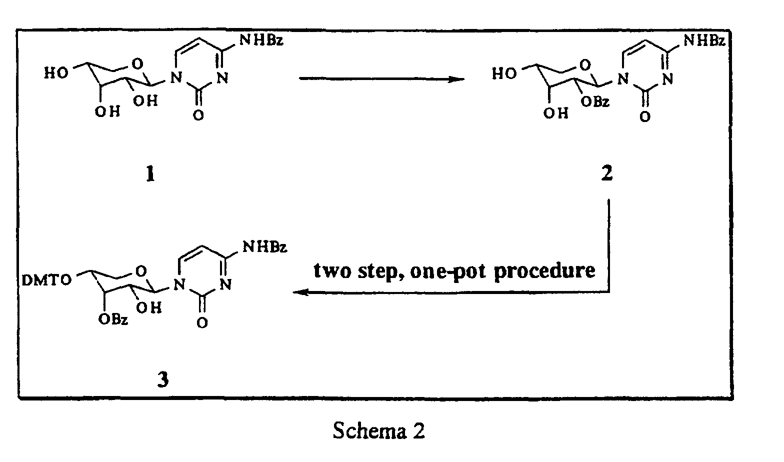

Alle Ansätze wurden unter N₂-Atmosphäre durchgeführt.

### N⁴-Benzoyl-1-(2'-O-benzoyl-β-D-ribo-pyranosyl)-cytosin 2:

54,0 g (0,155 mol) N⁴-Benzoyl-1-(β-D-ribo-pyranosyl)-cytosin **1** wurden in 830 ml Dimethylformamid (DMF) und 1,5 l Pyridin (beide Lösungsm. getrocknet und gelagert über Molsieb 3 Å) unter Erwärmen auf 124 °C gelöst. Bei -58° bis -63 °C wurden 23,0 g (0,163 mol; 1,05 eq.) BzCl, gelöst in 210 ml Pyridin, in 3,5 h zugetropft. Der Ansatz wurde im Kühlbad über Nacht gerührt. 90,3 g (1,5 mol; 10 eq.) n-Propanol wurden eingerührt und der Ansatz bei 40 °C im HV. eingeengt. Durch zweimalige Zugabe von 150 ml Toluol und erneutes Einengen wurden Pyridinreste entfernt. 124,3 g Rückstand wurden in 500 ml CH₂Cl₂ gelöst, zweimal mit je 300 ml halbkonzentrierter NaHCO₃-Lösung ausgerührt, und der ausgefallene Feststoff abfiltriert und getrocknet: 60,7 g Rückstand. Die CH₂Cl₂-Phase wurde eingeengt: 25,0 g. Getrennte Chromatographie an Kieselgel 60 (40 x 10 cm) mit Gradienten (AcOEt/iso-Hexan, 4:1, dann reines AcOEt, dann AcOEt/MeOH, 19:1 bis 2:1) lieferte (DC (Kieselgel, AcOEt)):

| | | | |
|---|---|---|---|
| 16,8 g | 2',4'-Dibenzoat | (24 %) | R_{f} 0,5. |
| 12,4 g | 1 | (23 %) | R_{f} 0,0. |
| 35,4 g | 2 | (51 %) | R_{f} 0,14. |

### N⁴-Benzoyl-1-{3'-O-benzoyl-4'-O-[(4,4'-dimethoxytriphenyl)-methyl]-β-D-ribopyranosyl}-cytosin 3:

35,4 g (78 mmol) **2** wurden in 390 ml CH₂Cl₂ und 180 ml Pyridin (beides wasserfrei) gelöst und 0,94 (7,8 mmol; 0,1 eq.) DMAP, 34,6 ml (203 mmol; 2,6 eq.) N-Ethyldiisopropylamin und 33,1 g (98 mmol; 1,25 eq.) DMTCI zugegeben und 2 h bei RT. gerührt.
DC (Kieselgel, AcOEt): R_{f} 0,6.
CH₂Cl₂ wurde bei 30 °C abgezogen, der Rückstand mit 640 ml Pyridin, 9,37 (78 mmol; 1,0 eq.) DMAP, 32,5 ml (234 mmol; 3,0 eq.) Et₃N, 21,7 g (156 mmol; 2,0 eq.) p-Nitrophenol und 93,8 g (1,56 mol; 20 eq.) n-Propanol versetzt und 42 h bei 65 °C gerührt. Der Ansatz wurde am HV. bei 50 °C eingeengt, zweimal mit je 250 ml Toluol versetzt und eingeengt.

Der Rückstand wurde in 1 l CH₂Cl₂ aufgenommen, dreimal mit je 500 ml verdünnter NaHCO₃-Lsg. ausgerührt, die org. Phase mit Na₂SO₄ getrocknet und eingeengt: 92,5 g Rückstand.
Chromatographie an Kieselgel 60 (50 x 10 cm) mit Gradienten (Methyl-tert. Butylether/iso-Hexan, 2:1 bis 4:1, dann Methyl-tert. Butylether/AcOEt, 1:4, dann AcOEt/MeOH, 1:1 bis 1:3) lieferte 44,7 g Produkt-haltige Fraktion, die aus 540 ml CH₂Cl₂/Methyl-tert. Butylether, 1:5, umkristallisiert wurde. Das Kristallisat wurde erneut aus 300 ml CH₂Cl₂/Methyl-tert. Butylether, 1:1, umkristallisiert.
3: DC (Kieselgel, CHCl₃/i-PrOH 49:1): R_{f} 0,14.

Man erhielt: 30,0 g N⁴-Benzoyl-1-{3'-O-benzoyl-4'-O-[(4,4'-dimethoxytriphenyl)-methyl]-β-D-ribo-pyranosyl}-cytosin 3
d. s. 51 % Ausbeute bezogen auf **2**. ¹H-NMR entspricht.

### Beispiel 3

### Synthese des N⁶-Benzoyl-9-{3'-O-benzoyl-4'-O-[(4,4-dimethoxytriphenyl)-methyl]-β-Dribo-pyranosyl}-adenins

Zuerst 2'-Substitution, dann 4'-Substitution, dann Wanderungsreaktion:

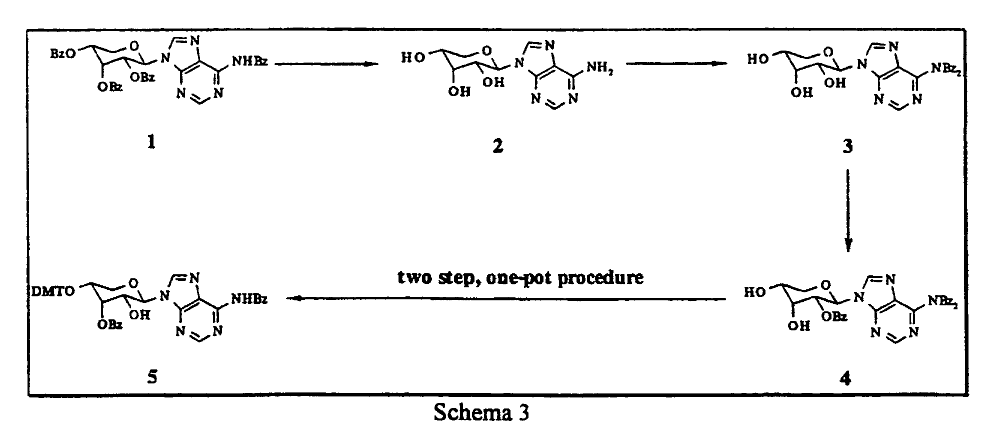

### 9-(β-D-Ribo-pyranosyl)-adenin 2:

68,37 g (100 mmol) N⁶-Benzoyl-9-(2',3',4'-tri-O-benzoyl-β-D-ribo-pyranosyl)-adenin **1** wurde in 300 ml NH₃-gesättigtem MeOH über Nacht bei RT. gerührt und das Kristallisat abfiltriert: 23,5 g (88 %) **2.**
DC (Kieselgel, AcOEt/MeOH 2:1): R_{f} 0,23.
^{**1**}**H-NMR** (300 MHz, DMSO): 3,56-3,78 (m, 3H, H(4'), H(5')); 4,04 (m, 1H, H(3')); 4.23 (ddd, J = 2,5, 8, 9,5 Hz, H(2')), 4,89 (d, J = 6 Hz, 1H, OH), 5,07 (d, J = 7 Hz, 1H, OH), 5,12 (d, J = 4 Hz, 1H, OH), 5,63 (d, J = 9,5 Hz, 1H, H(1')), 7,22 (s, 2H, NH2), 8,14 (s, 1H, H(2)), 8,29 (s, 1H, H(8)).
^{**13**}**C-NMR** (75 MHz, DMSO): 65,0 (t, C(5')); 66,6 (s, C(4')), 68,1 (s, C(3')), 71,1 (s, C(2')), 79,6 (s, C(1')); 118.6 (C(5)); 139,5 (s, C(8)), 149,9 (s, C(4)), 152,5 (s, C(2)), 155,8 (s, C(6)).

### N⁶,N⁶-Dibenzoyl-9-(β-D-ribo-pyranosyl)-adenin 3:

Unter N₂-Atmosphäre wurden 16,8 g (62,9 mmol) 2 in 500 ml wasserfreiem Pyridin suspendiert und auf -4 bis - 10 °C gekühlt. Innerhalb von 20 min wurden 40 ml (199 mmol; 5 eq.) Trimethylchlorsilan zugetropft und 2,5 h unter Kühlung gerührt.
Bei -10 bis -15 °C wurden 36,5 ml (199 mmol; 5 eq.) Benzoylchlorid, gelöst in 73 ml Pyridin, innerhalb von 25 min zugesetzt, 10 min unter Kühlung und 2 h bei RT. gerührt (DC-Kontrolle (Kieselgel, AcOEt/Heptan 1:1): R_{f} 0,5). Man kühlte wieder auf -10 °C, ließ 136 ml H₂O (Temp. max. +8 °C) zulaufen und rührte über Nacht bei RT. Nach vollständigem Umsatz wurde das Lösungsmittel abgezogen und der Rückstand zweimal in je 200 ml Toluol aufgenommen und wieder eingedampft. Man versetzte mit je 500 ml Et₂O und H₂O, ließ mechanisch 2 h rühren, filtrierte das in beiden Phasen nur wenig lösliche Produkt ab, wusch mit Et₂O und H₂O und trocknete über P₂O₅ am HV.: 23,8 g (80 %) **3.**
DC (Kieselgel, AcOEt/MeOH 9:1): R_{f} 0,35.
^{**1**}**H-NMR** (300 MHz, DMSO): 3,60-3,80 (m, 3H, H(4'), H(5')); 4,06 (bs, 1H, H(3')); 4.30 (ddd, J = 2,5, 8, 9,5 Hz, H(2')), 4,93 (d, J = 6 Hz, 1H, OH), 5,20 (d, J = 4 Hz, 1H, OH), 5,25 (d, J = 4 Hz, 1H, OH), 5,77 (d, J = 9,5 Hz, 1H, H(1')), 7,47 (m, 4 H, Harom), 7,60 (m, 2H, Harom), 7,78 (m, 4H, Harom), 8,70 (s, 1H, H-C(2), 8,79 (s, 1H, H(8)).
^{**13**}**C-NMR** (75 MHz, DMSO): 66,2 (t, C(5')); 66,5 (s, C(4')), 68,0 (s, C(3')), 71,0 (s, C(2')), 80,4 (s, C(1')); 112.42 (C(5)); 126,9 (s, C(5)), 126,9, 128,9, 133,3, 133,4 (arom. C), 146,0 (s, C(8)), 150,7 (s, C(4)), 151,8 (s, C(2)), 153,3 (s, C(6)), 172,0 (s, C=O)).

### N⁶, N⁶-Dibenzoyl-9-(2'-O-benzoyl-β-D-ribo-pyranosyl}-adenin 4:

Unter N₂-Atmosphäre wurden 26,4 g (55,5 mmol) 3 in 550 ml wasserfreiem CH₂Cl₂ und 55 ml Pyridin (jeweils gelagert über Molsieb) gelöst, mit 0,73 g (5,55 mmol; 0,1 eq.) DMAP versetzt und auf -87 bis -90 °C abgekühlt. Innerhalb von 1 h wurden 8,58 g (61 mmol; 1,1 eq.) BzCl in 14 ml Pyridin zugetropft und über 60 h (Wochenende) bei -78 °C belassen. Der Ansatz wurde eingeengt, zweimal mit je 100 ml Toluol versetzt und eingedampft, um Pyridin zu entfernen. Chromatographie an Kieselgel 60 (20 x 10 cm) mit Gradienten (AcOEt/Heptan, 1:1 bis 9:1) lieferte 23,2 g **4.**
**4:** DC (Kieselgel, AcOEt): R_{f} 0,34.

### N⁶-Benzoyl-9-{3'-O-benzoyl-4'-O-[(4,4'-dimethoxytriphenyl)-methyl]-β-D-ribo-pyranosyl}-adenin 5:

23,2 g (40 mmol) 4 wurden in 160 ml wasserfreiem CH₂Cl₂ gelöst und in Folge mit 14,9 g (56 mmol; 1,1 eq.) DMTCl und 17,7 ml (104 mmol; 2,6 eq.) N-Ethyldiisopropylamin versetzt. Nach 2 h Rühren bei RT wurden weitere 4,0 g (11,8 mmol; 0,3 eq.) DMTCI zugefügt und weitere 40 min gerührt. Der Ansatz wurde bei 350-520 mbar und 35 °C am Rotavapor eingeengt.
DC (Kieselgel, AcOEt/Heptan 1:1): R_{f} 0,18.
Der Rückstand wurde in 260 ml wasserfreiem Pyridin gelöst und in Folge mit 51 ml (679 mmol; 17 eq.) n-Propanol, 16,6 ml (120 mmol; 3 eq.) Et₃N, 11,1 g (80 mmol; 2 eq.) p-Nitrophenol und 5,3 g (44 mmol; 1,1 eq.) DMAP versetzt und bei 60-63 °C 23 h lang gerührt. Dann blieb der Ansatz bei RT. 21 h stehen. Das Reaktionsgemisch wurde am Rotavapor eingeengt. Der Rückstand wurde zweimal mit je 200 ml Toluol versetzt und eingeengt, in CH₂Cl₂ gelöst und dreimal mit Wasser extrahiert.
Chromatographie an Kieselgel 60 (30 x 10 cm) mit Gradienten (AcOEt/Heptan, 1:2 bis 1:0; dann AcOEt/MeOH, 1:0 bis 9:1) lieferte 13 g 5.
**5:** DC (Kieselgel, AcOEt/Heptan 4:1): R_{f} 0,2.

Man erhielt: 13 g N⁶-Benzoyl-9-{3'-O-benzoyl-4'-O-[(4,4'-dimethoxytriphenyl)-methyl]-β-D-ribo-pyranosyl}-adenin 5
d. s. 30 % Ausbeute bezogen auf **3.** ¹H-NMR entspricht.
Zeitersparnis gegenüber literaturbekanntem Verfahren: 50 %.

### Beispiel 4

### Synthese von 9-[3'-O-Benzoyl-4'-O-((4,4'-dimethoxytriphenyl)-methyl)-β-Dribopyranosyl]-2-O-allyl-2-N-isobutyroyl-guanin

Zuerst 3'-Substitution, dann 4'-Substitution:

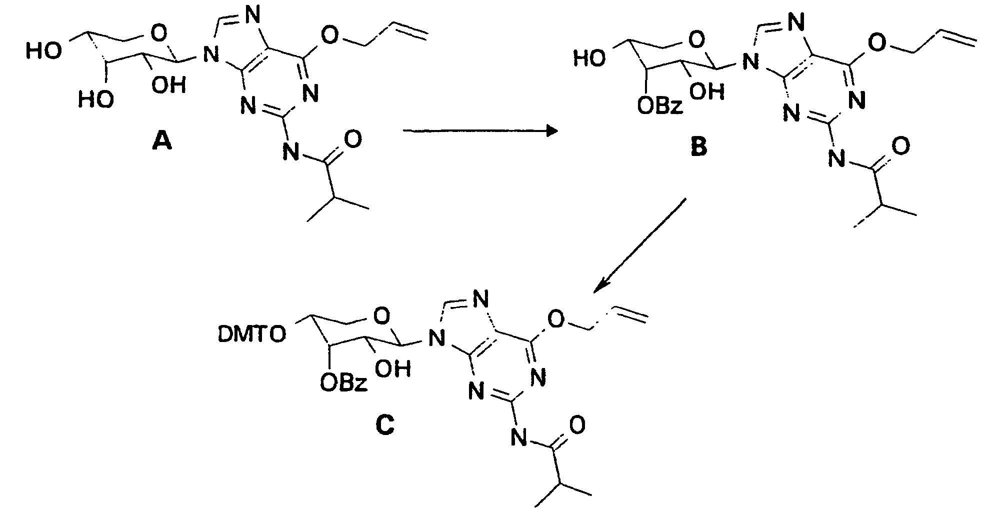

### 9-[3'-O-Benzoyl-β-D-ribopyranosyl]-2-O-allyl-2-N-isobutyroyl-guanin B

Das G-Triol **A** (393 mg, 1.0 mmol) wurde in 4 ml trockenem Dichlormethan gelöst. Man versetzte mit Benzoesäuretrimethylorthoester (0.52 ml, 3.0 mmol) und Camphersulfonsäure (58 mg, 0.25 mmol) und rührte 15 h bei Raumtemperatur. Dann wurde die Mischung auf 0 °C abgekühlt und mit 2 ml einer auf 0 °C vorgekühlten Mischung von Acetonitril, Wasser und Trifluoressigsäure (50:5:1) versetzt. Man rührte 10 min und entfernte das Lösungsmittel im Vakuum. Der Rückstand wurde durch Flashchromatographie an Kieselgel (2.3 x 21 cm) mit Dichlormethan/Methanol 100:3 gereinigt. Man erhielt 25 mg (5%) 4-O-Benzoylverbindung 139 mg (28%) Mischfraktionen und 205 mg (41 %) der gewünschten 3-O-Benzoylverbindung **B.**
¹H-NMR (300 MHz, CDCl₃): 1.12, 1.14 (2 d, J = 7.0 Hz, 2 x 3 H, NHCOCHMe₂), 2.78 (hep, J = 7 Hz, 1 H, NHCOCHMe₂), 3.85 (dd, J = 6.0, 11.0 Hz, 1 H, H-5'_{eq}), 3.94 (app. T, J = 11.0 Hz, 1 H, H-5'ₐₓ), 4.12 (ddd, J = 2.5, 6.0, 11.0 Hz, 1 H, H-4'), 4.52 (dd, J = 3.5, 9.5 hz, 1 H, H-2'), 5.00 (dt, J = 1.5, 6.0 Hz, 2 H, All), 5.19 (dq, J = 1.5, 10.0 Hz, 1 H, All), 5.39 (dq, 1.5, 16.5 Hz, 1 H, All), 5.85 (bt, J = 3.0 Hz, 1 H, H-3'), 5.97 (d, J = 9.5 Hz, 1 H, H-1'), 6.07 (ddd, J = 6.0, 10.0, 16.5 Hz, 1 H, All), 7.40-7.58 (m, 3 H, Bz), 8.10-8.16 (m, 2 H, Bz), 8.28 (s, 1 H, H-8).

### 9-[3'-O-Benzoyl-4'-O-((4,4'-dimethoxytriphenyl)-methyl)-β-D-ribopyranosyl]-2-Oallyl-2-N-isobutyroyl-guanin C

Das Diol **B** (101 mg, 0.2 mmol) wurde in 3.2 ml trockenem Dichlormethan suspendiert. Man versetzte mit 171 µl (1.0 mmol) N-Ethyldiisopropylamin, 320 µl (3.96 mmol) Pyridin und 102 mg (0.3 mmol) DMTCI und rührte bei Raumtemperatur. Nach 24 h wurden weitere 102 mg (0.3 mmol) DMTCl zugegeben und nochmals 24 h gerührt. Dann wurde mit 30 ml Dichlormethan verdünnt. Die Lösung wurde mit 20 ml 10%iger wässriger Citronensäurelösung und 10 ml gesättigter Natriumbicarbonatlösung gewaschen, über MgSO₄ getrocknet und im Vakuum eingeengt. Der Rückstand wurde durch Flashchromatographie an Kieselgel (2.3 x 20 cm) mit Dichlormethan/Methanol 100:1 gereinigt. Man erhielt 39 mg des bekannten, gewünschten Produkts ***C*** (24%).

### Beispiel 5

### Synthese von p-RNA-Linkersystemen

Im folgenden werden drei Wege beschrieben, die die Bereitstellung von Linkern, die einen Amino-Terminus aufweisen, der dann zum Anlinken funktioneller Einheiten verwendet werden kann, ermöglichen:

### 5.1 Uracil-basierender Linker

### auf der Basis der Modifizierung der 5-Position des Uracils.

Die Herstellung von Hydroxyethyluracil **28** gelingt im großen Maßstab nach bekannter Methode (J.D. Fissekis, A. Myles, G.B. Brown, J. Org. Chem. **1964,** 29, 2670) g-Butyrolacton **25** wurde mit Ameisensäuremethylester formyliert, das Natriumsalz **26** zum Harnstoffderivat **27** umgesetzt und dieses zum Hydroxyethyluracil **28** cyclisiert (Schema 4).

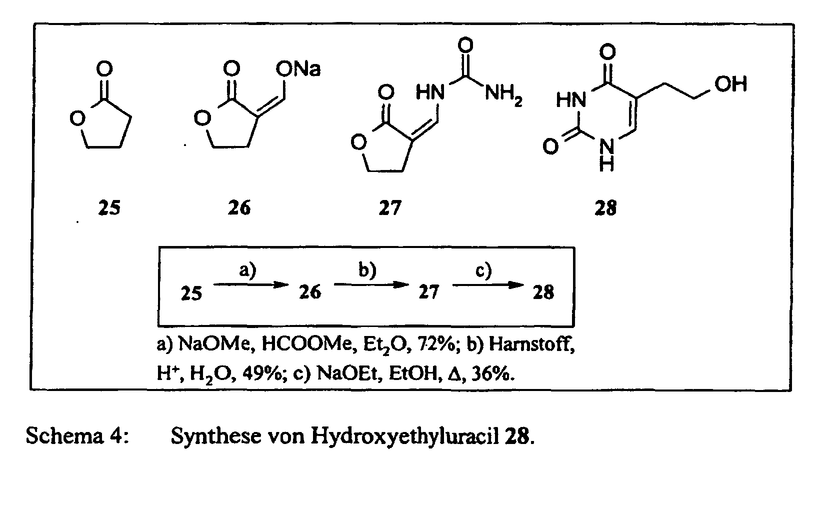

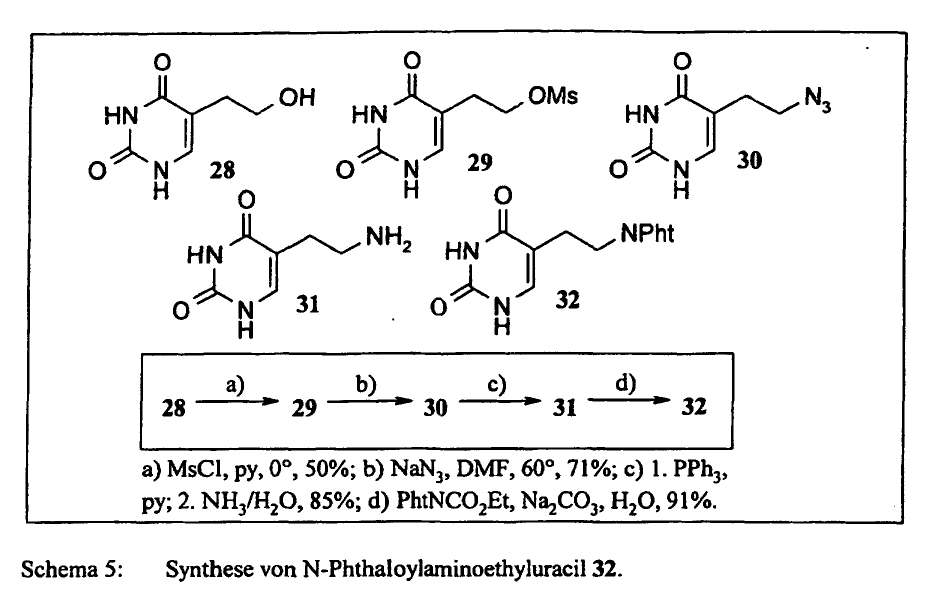

### Hydroxyethyluracil 28 wurde mit Methansulfonsäurechlorid in Pyridin mesyliert zu 29 (J.D. Fissekis, F. Sweet, J. Org. Chem.1973, 38, 264).

Die folgenden Stufen wurden neu erfunden: Mit Natriumazid in DMF wurde **29** zum Azid **30** umgesetzt und dieses mit Triphenylphosphin in Pyridin zum Aminoethyluracil **31** reduziert. Die Aminofunktion in **31** wurde schließlich mit N-Ethoxycarbonylphtalimid geschützt (Schema 5). Nukleosidierung von Ribosetetrabenzoat **33** mit N-Phtaloylaminoethyluracil **32** lieferte das Ribosetribenzoat **34** in guten Ausbeuten. Das anomere Zentrum des Pyranoseringes ist, wie aus der Kopplungskonstanten zwischen H-C(1') und H-C(2') von J = 9.5 Hz klar ersichtlich, β-konfiguriert. Anschließende Abspaltung der Benzoatschutzgruppen mit NaOMe in MeOH lieferte das Linkertriol **35. 35** wurde bei - 78°C in Pyridin/Dichlormethan 1:10 in Gegenwart von DMAP mit Benzoylchlorid umgesetzt. Dabei erhielt man neben dem gewünschten 2'-Benzoat **36** (64%) auch 2',4'dibenzoyliertes Produkt (22%), welches gesammelt und analog der Methanolyse von **34** nach **35** wieder in das Triol **35** umgewandelt wurde. Das 2'-Benzoat **36** wurde mit Dimethoxytritylchlorid in Gegenwart von Hünig-Base in Dichlormethan in 4'-Position in Ausbeuten größer 90% trityliert. Die Umlagerung von 4'-DMT-2'-benzoat **37** zum 4'-DMT-3'-benzoat **38** erfolgte in Gegenwart von DMAP, p-Nitrophenol und Hünig-Base in n-Propanol/Pyridin 5:2. Nach Chromatographie wird **38** erhalten. 4'-DMT-3'-benzoat **38** wurde abschließend mit ClP(OAll)N(iPr)₂ in Gegenwart von Hünig-Base zum Phosphoramidit **39** umgesetzt (Schema 6). Dieser läßt sich ohne Änderung der Syntheseprotokolle für die automatisierte Oligonucleotidsynthese einsetzen.

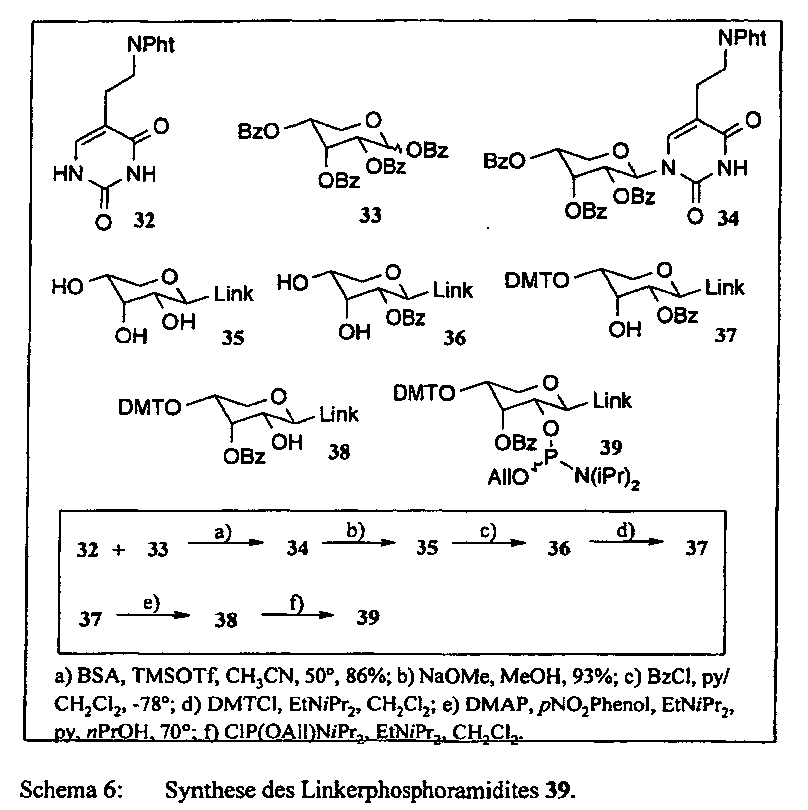

### Ausführung:

### Synthese eines Uracil-Linker-Bausteins

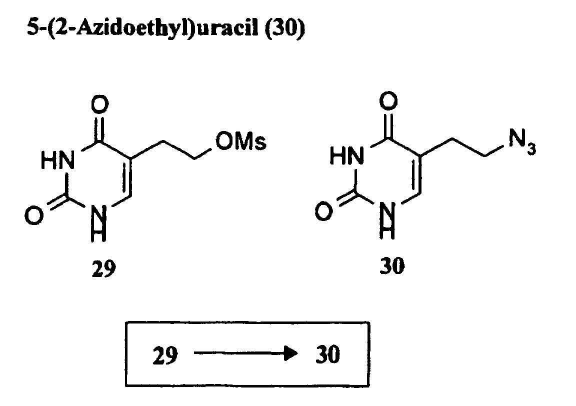

### 1. Durchführung

In einem mit Innenthermometer und Rückflußkühler ausgestatteten 500-ml-Dreihalskolben wurden 26.0 g (0.11 mol) 29 in 250 ml DMF gelöst und mit 10.8 g (0.166 mol) Natriumazid versetzt. Die Suspension wurde im Anschluß vier Stunden bei 60°C gerührt (DC-Kontrolle, CHCl₃/MeOH 9:1). Das DMF wurde abdestilliert und der Rückstand mit 150 ml Wasser verrührt. Der Feststoff wurde abfiltriert, mit ca. 50 ml Wasser gewaschen und über Nacht im Vakuum im Exsiccator über Phosphorpentoxid getrocknet. Man erhielt 14.2 g (71%) 30 in Form eines farblosen Feststoffes vom Schmp. 230-235°C (u. Zers.).

### 2. Analytische Daten

### 5-(2-Azidoethyl)uracil (30):

- **Schmp.:**: 230-235°C u. Zersetz..
- **DC:**: CHCl₃/MeOH 9:1, R_{f} 0.48.
- **UV** (MeOH):: λₘₐₓ 263.0 (7910).
- **IR** (KBr):: 3209s, 3038s, 2139s, 1741s, 1671s, 1452m, 1245m, 1210m.
- ^{**1**}**H-NMR** (300 MHz, d₆-DMSO):: 2.46 (t, 2H, J(CH₂CH₂N, CH₂CH₂N) = 7.0, CH₂CH₂N); 3.40 (t, 2H, J(CH₂CH₂N, CH₂CH₂N) = 7.0, CH₂CH₂N); 7.36 (s, H-C(6)); 11.00 (br. s, 2H, H-N(1), H-N(3)).
- **MS** (ESI⁺):: 180.0[M+H]..

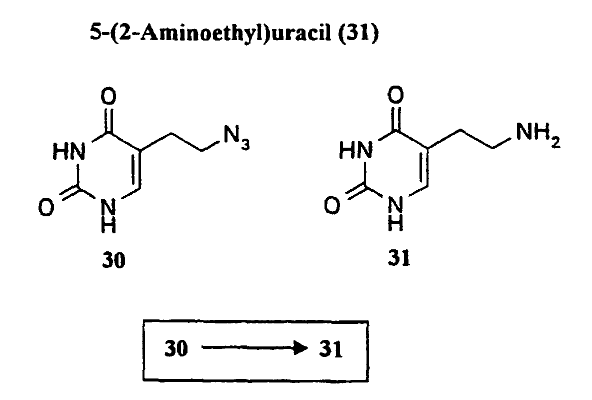

### 1. Durchführung

In einem mit Innenthermometer und Rückflußkühler ausgestatteten 250-ml-Dreihals-kolben wurden 14.2 g (78.0 mmol) **30** in 175 ml Pyridin suspendiert und mit 61.4 g (234 mmol) Triphenylphosphin versetzt²⁾. Es wurde fünf Stunden auf 60°C erwärmt und über Nacht bei Raumtemp. gerührt (DC-Kontrolle, CHCl₃/MeOH 5:1). Zur Suspension wurden 40 ml einer 25%igen Ammoniaklösung gegeben, die daraufhin aufklarte. Die Lösungsmittel wurden i.v.i.RV entfernt. Der Rückstand wurde in 200 ml CH₂Cl₂/MeOH 1:1 30 min bei Raumtemp. gerührt, der Niederschlag abfiltriert und mit CH₂Cl₂ gewaschen. Nach Trocknen im Vakuum im Exsiccator über Phosphorpentoxid erhielt man 10.0 g (85%) **31** vom Schmp. 214-220°C.

### 2. Analytische Daten

### 5-(2-Aminoethyl)uracil (31):

- **Schmp.:**: 214-220°C u. Gasentwicklung, vorher sintern.
- **DC:**: CHCl₃/MeOH/HOAc/H₂O 85:20:10:2, R_{f} 0.07.
- **UV** (MeOH):: λₘₐₓ 263.0 (6400).
- **IR** (KBr):: 3430m, 3109s, 1628s, 1474m, 1394s, 1270s, 1176w, 1103m, 1021m, 966m, 908m, 838m.
- ^{**1**}**H-NMR** (300 MHz, d₆-DMSO):: 2.21 (t, 2H, J(CH₂CH₂N, CH₂CH₂N) = 6.8, CH₂CH₂N); 2.59 (t, 2H, J(CH₂CH₂N, CH₂CH₂N) = 6.8, CH₂CH₂N); 5.90 (v. br. s, 4H, H-N(1), H-N(3), NH₂); 7.19 (s, H-C(6)).
- **MS** (ESI⁻):: 153.9 [M-H].

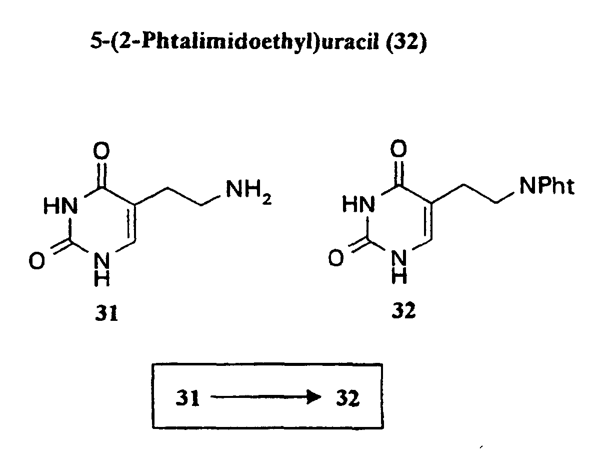

### 1. Durchführung

In einem 250-ml-Rundkolben wurden 9.6 g (61.8 mmol) **31** in 100 ml Wasser suspendiert und mit 6.64 g (62.6 mmol) Na₂CO₃ versetzt. Nach 15 min Rühren bei Raumtemp. gab man portionsweise 14.3 g (65 mmol) N-Ethoxycarbonylphtalimid zu und rührte drei Stunden bei Raumtemp. (DC-Kontrolle, CHCl₃/MeOH 5:1). Die nunmehr zähe, weiße Suspension wurde vorsichtig¹⁾ mit konz. Salzsäure auf pH 4 eingestellt und der weiße Niederschlag abfiltriert. Nach Waschen mit Wasser wurde der Feststoff im Vakuum im Exsiccator über Phosphorpentoxid getrocknet. Dies lieferte 16.0 g (91%) **32** vom Schmp. 324-327°C.

### 2. Analytische Daten

### 5-(2-Phtalimidoethyl)uracil (32):

- **Schmp.:**: 324-327°C u. Zersetz..
- **DC:**: CHCl₃/MeOH 5:1, R_{f} 0.51.
- **UV** (MeOH):: λₘₐₓ 263.0 (5825); λ 298.0 (sh., 1380).
- **IR** (KBr):: 3446m, 3216m, 1772m, 1721s, 1707s, 1670s, 1390m.
- ^{**1**}**H-NMR** (300 MHz, d₆-DMSO):: 2.49 (t, 2H, J(CH₂CH₂N, CH₂CH₂N) = 6.0, CH₂CH₂N); 3.71 (t, 2H, J(CH₂CH₂N, CH₂CH₂N) = 6.0, CH₂CH₂N); 7.24 (s, H-C(6)); 7.84 (m_{c}, 4H, NPht); 10.76 (br. s, H-N(1), H-N(3)).
- **MS** (ESI⁻):: 284.0 [M-H].

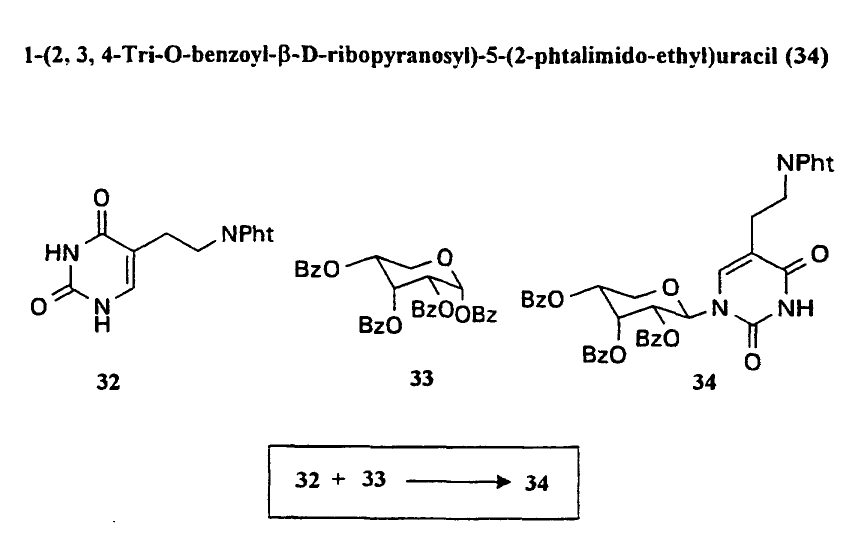

### 1. Durchführung

In einem 250-ml-Dreihalskolben, ausgestattet mit Argonüberleitung, Innenthermometer und Septum, wurden 7.00 g (24 mmol) **32** und 13.6 g (24 mmol) **33** in 120 ml Acetonitril suspendiert. Dann wurden mittels Spritze zunächst 12.2 ml (50 mmol) BSA zugegeben und nach 30 min Rühren nochmals 7 ml (28 mmol) BSA zugegeben. Nach kurzzeitigem Erwärmen auf 40°C klarte sich die Reaktionsmischung auf. Bei Raumtemp. wurden 13 ml (72 mmol) TMSOTf mittels Spritze zugegeben. Nach einer Stunde konnte noch keine Produktbildung beobachtet werden (DC-Kontrolle, AcOEt/n-Heptan 1:1). Daher wurden weitere 13 ml (72 mmol) TMSOTf zugegeben. Im Anschluß wurde der Reaktionsansatz auf 50°C erwärmt. Nach 2.5 h Rühren bei 50°C (DC-Kontrolle) wurde auf RT. gekühlt, auf eine eiskalte Mischung von 250 ml AcOEt und 190 ml ges. NaHCO₃-Lösung und 10 min intensiv ausgerührt. Man wusch nochmals mit 100 ml NaHCO₃-Lösung und extrahierte die wäßrigen Phasen nochmals mit 100 ml AcOEt. Die ver. org. Phasen wurden mit MgSO₄ getrocknet und die Lösungsmittel i.V.i.RV entfernt. Nach Trocknen im ÖPV erhielt man 20.9 g Rohprodukt. Chromatographie an Kieselgel (h = 25 cm, ϕ = 5 cm, AcOEt/n-Heptan 1:1) lieferte ein DC-einheitliches, schaumiges Produkt, das mit Et₂O digeriert wurde. Filtration und Trocknen im ÖPV ergab 15 g (86%) **34.**

### 2. Analytische Daten

### 1-(2, 3, 4-Tri-O-benzoyl-β-D-ribopyranosyl)-5-(2-phtalimidoethyl)uracil (34):

- **Schmp.:**: 124°C (sintern).
- **DC:**: AcOEt/n-Heptan 1:1, R_{f} 0.09.
- **UV** (MeOH):: λₘₐₓ 263.0 (11085); λ 299.0 (sh., 1530).
- **IR** (KBr):: 3238w, 3067w, 1772m, 1710s, 1452m, 1395m, 1266s, 1110s, 1070m, 1026m.
- ^{**1**}**H-NMR** (300 MHz, CDCl₃):: 2.79 (m_{c}, 2H, CH₂CH₂N); 3.96 (m_{c}, 2H, CH₂CH₂N); 4.06 (dd, J(H_{eq}-C(5'), Hₐₓ-C(5')) = 11.0, J(H_{eq}-C(5'), H-C(4')) = 6.0, H_{eq}-C(5')); 4.12 (t, J(Hₐₓ-C(5'), H_{eq}-C(5')) = J(Hₐₓ-C(5'), H-C(4')) = 11.0, Hₐₓ-C(5')); 5.39 (dd, J(H-C(2'), H-C(1')) = 9.5, J(H-C(2'), H-C(3')) = 2.9, H-C(2')); 5.46 (ddd, J(H-C(4'), Hₐₓ-C(5')) = 11.0, J(H-C(4'), H_{eq}-C(5')) = 6.0, J(H-C(4'), H-C(3')) = 2.9, H-C(4')); 6.26 (ψt, J ≈ 2.6, H-C(3')); 6.36 (d, J(H-C(1'), H-C(2')) = 9.5, H-C(1')); 7.24-7.40, 7.44-7.56, 7.61-7.66, 7.72-7.80, 7.84-7.90, 8.06-8.13 (6m, 16H, 3 Ph, H-C(6)); 7.70, 7.82 (2 m_{c}, 4H, NPht); 8.37 (s, H-N(3)).
- ^{**13**}**C-NMR** (75 MHz, CDCl₃):: 21.19 (CH₂CH₂N); 36.33 (CH₂CH₂N); 64.07 (C(5')); 66.81, 68.22 (C(4'), C(2')); 69.29 (C(3')); 78.59 (C(1')); 112.42 (C(5)); 123.31, 132.05, 133.89 (6C, Pht); 128.33, 128.47, 128.47, 128.83, 128.86, 129.31, 129.83, 129.83, 129.94, 133.55, 133.62, 133.69 (18C, 3 Ph); 135.87 (C(6)); 150.39 (C(2)); 162.78 (C(4)); 164.64, 165.01, 165.41 (3C, O₂CPh); 168.43 (2C, CO-Pht).
- **MS** (ESI⁺):: 730.2 [M+H].

| | | | | |
|---|---|---|---|---|
| **Anal.** | ber. für C₄₀H₃₁N₃O₁₁ (729.70) | C 65.84, | H 4.28, | N 5.76; |
| | gef. | C 65.63, | H 4.46, | N 5.53. |

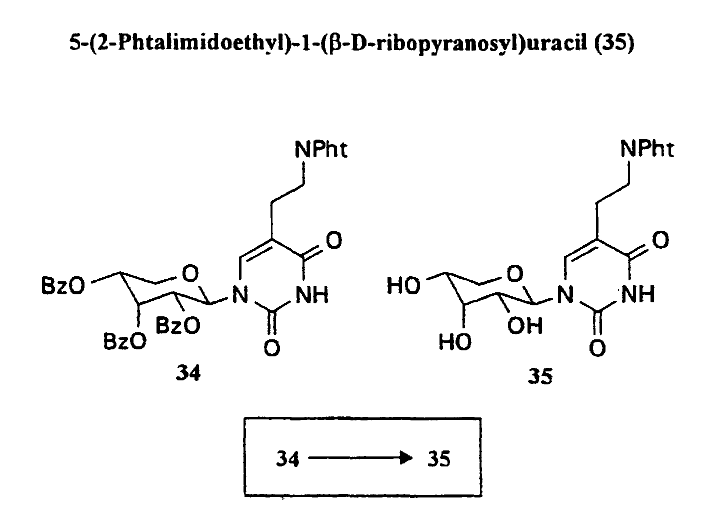

### 1. Durchführung

In einem 1-1-Rundkolben wurden 15 g (20 mmol) 34 in 500 ml MeOH gelöst, mit 324 mg (6 mmol) NaOMe versetzt und über Nacht unter Wasserausschluß bei Raum-temp. gerührt (DC-Kontrolle, AcOEt/n-Heptan 1:1). Es wurde solange Amberlite IR-120 zur entstandenen Suspension gegeben bis der pH-Wert <7 war. Es wurde der Feststoff in der Hitze gelöst, heiß vom Ionentauscher abfiltriert und mit MeOH gewaschen. Nach Entfernen der Lösungsmittel wurde der Rückstand zweimal mit je 150 ml Wasser coevaporiert. Dies lieferte 9 g Rohprodukt, das 10 min in 90 ml MeOH unter Rückfluß erhitzt wurde. Nach Abkühlen auf Raumtemp. versetzte man mit 60 ml Et₂O und bewahrte über Nacht bei 4°C auf. Filtration, Waschen mit Et₂O und Trocknen im ÖPV lieferte 7.8 g (93%) **35**.

### 2. Analytische Daten

### 5-(2-Phtalimidoethyl)-1-(β-D-ribopyranosyl)uracil (35):

- **Schmp.:**: 137°C (sintern).
- **DC:**: CHCl₃/MeOH 5:1, R_{f} 0.21.
- **UV** (MeOH):: λₘₐₓ 263.0 (8575); λ 299.0 (sh., 1545).
- **IR** (KBr):: 3458s, 1772w, 1706s, 1400m, 1364m, 1304m, 1045m.
- ^{**1**}**H-NMR** (300 MHz, d₆-DMSO + 2 Tr. D₂O):: 2.55 (m_{c}, 2H, CH₂CH₂N); 3.28-3.61 (m, 4H, H-C(2'), H-C(4'), H_{eq}-C(5'), Hₐₓ-C(5')); 3.73 (m_{c}, 2H, CH₂CH₂N); 3.93 (m, H-C(3')); 5.50 (d, J(H-C(1'), H-C(2')) = 9.3, H-C(1')); 7.41 (s, H-C(6)); 7.84 (s, 4H, NPht).
- ^{**13**}**C-NMR** (75 MHz, d₆-DMSO):: 25.63 (CH₂CH₂N); 36.62 (CH₂CH₂N); 64.95 (C(5')); 66.29 (C(4')); 67.37 (C(2')); 71.12 (C(3')); 79.34 (C(1')); 110.39 (C(5)); 122.85, 131.54, 134.08 (6C, Pht); 137.92 (C(6)); 150.84 (C(2)); 163.18 (C(4)); 167.74 (2C, CO-Pht).
- **MS** (ESI⁻):: 416.1 [M-H].

### 1-(2'-O-Benzoyl-β-D-ribopyranosyl)-5-(2-phtalimidoethyl)-uracil

In einem ausgeheizten und mit Argon begasten 1-I-Vierhalskolben wurden 10.6 g (0.025 mmol) 5-(2-Phtalimidoethyl)-1-(β-D-ribopyranosyl)uracil in 20 ml Pyridin gelöst und mit 200 ml Dichlormethan versetzt. Es wurde auf -70°C abgekühlt, unter Kühlung 3.82 ml (0.033 mmol) Benzoylchlorid in 5 ml Pyridin und 20 ml Dichlormethan langsam zugetropft und 35 min bei -70°C gerührt. Das Reaktionsgemisch wurde auf 600 ml gekühlte Ammoniumchlorid-Lösung gegossen und die wäßrige Phase mit Ethylacetat extrahiert. Die vereinigten organischen Phasen wurden mit Wasser gewaschen, getrocknet und im Vakuum zur Trockene eingeengt. Chromatographie über Kieselgel (Ethylacetat/Heptan 1:1) lieferte 7.9 g (60%) 1-(2'-O-Benzoyl-β-D-ribopyranosyl)-5-(2-phtalimidoethyl)-uracil.
DC: R_{f} 0.24 (Ethylacetat/Heptan 4:1).
¹H-NMR (300 Mhz, d₆-DMSO): 2.67 (m_{c}, 2H, CH₂CH₂N); 3.66-3.98 (m, 5H, H-C(4'), H_{eq}-C(5'), Hₐₓ-C(5'), CH₂CH₂N); 4.51 (t, 1H, H-C(3')); 4.98 (dd, 1H, H-C(2')); 6.12 (d, 1H, H-C(1')); 7.19 (s, 1H, H-C(6)); 7.29-7.92 (m, 9H, OBz, NPht).

### 1-(2-O-Benzoyl-4-O-(4,4'-dimethoxytrityl)-β-D-ribopyranosyl)-5-(2-phtalimidoethyl)uracil

5.6 g (10.73 mmol) 1-(2-O-Benzoyl-β-D-ribopyranosyl)-5-(2-phtalimidoethyl)uracil wurden in 60 ml Dichlormethan gelöst, mit 4.72 g (13.95 mmol) 4,4'-Dimethoxytritylchlorid und 2.4 ml (13.95 mmol) N-Ethyl-diisopropylamin versetzt und 20 min bei RT gerührt. Das Reaktionsgemisch wurde mit 100 ml Dichlormethan verdünnt, mit Natriumhydrogencarbonat-Lösung und 20% Zitronensäure-Lösung gewaschen, getrocknet und im Vakuum zur Trockene eingeengt. Chromatographie über Kieselgel (Ethylacetat/Heptan 1:1 + 2% Triethylamin) lieferte 7.7 g (87%) 1-(2-O-Benzoyl-4-O-(4,4'-dimethoxytrityl)-β-D-ribopyranosyl)-5-(2-phtalimidoethyl)uracil.
DC: R_{f} 0.53 (Ethylacetat/Heptan 1:1 + 2% Triethylamin).
¹H-NMR (300 MHz, CDCl₃): 2.64 (m_{c}, 2H, CH₂CH₂N); 3.12 (m_{c}, 1H, H-C(4')); 3.59-3.63 und 3.72-3.92 (m, 5H, H-C(3'), H_{eq}-C(5'), Hₐₓ-C(5'), CH₂CH₂N); 3.81 und 3.82 (s, 6H, CH₃O); 4.70 (dd, 1H, H-C(2')); 6.09 (d, 1H, H-C(1')); 7.05 (s, 1H, H-C(6)); 6.84-7.90 (m, 22H, ODmt, OBz, NPht).

### 1-(3-O-Benzoyl-4-O-(4,4'-dimethoxytrityl)-β-D-ribopyranosyl)-5-(2-phtalimidoethyl)uracil

3 g (3.63 mmol) 1-(2-O-Benzoyl-4-O-(4,4'-dimethoxytrityl)-β-D-ribopyranosyl)-5-(2-phtalimidoethyl)uracil, 1 g (7.26 mmol) 4-Nitrophenol, 0.44 g (3.63 mmol) 4-(Dimethylamino)pyridin und 3.75 ml (21.78 mmol) N-Ethyl-diisopropylamin wurden in 5.6 ml iso-Propanol und 25 ml Pyridin gelöst, auf 65°C erhitzt und 3 Tage bei 65°C gerührt. Die Lösung wurde im Vakuum zur Trockene eingeengt und der Rückstand in 150 ml Dichlormethan gelöst. Nach Waschen mit 20% Zitronensäure-Lösung und Natriumhydrogencarbonat-Lösung wurde über Magnesiumsulfat getrocknet. Chromatographie über Kieselgel (Ethylacetat/Dichlormethan/iso-Hexan 2:1:1) lieferte 2.27 g (76%) 1-(3-O-Benzoyl-4-O-(4,4'-dimethoxytrityl)-β-D-ribopyranosyl)-5-(2-phtalimidoethyl)uracil.
DC: R_{f} 0.27 (Ethylacetat/iso-Hexan 2:1 + 1% Triethylamin).
¹H-NMR (300 MHz, CDCl₃): 2.39 (m_{c}, 2H, CH₂CH₂N); 2.53 (m_{c}, 1H, H_{eq}-C(5')); 3.30 (dd, 1H, H-C(2')); 3.55 (m_{c}, 1H, Hₐₓ-C(5')); 3.69 (m_{c}, 2H, CH₂CH₂N); 3.78 und 3.79 (s, 6H, CH₃O); 3.79-3.87 (m, 1H, H-C(4')); 5.74 (d, 1H, H-C(1')); 5.77 (m_{c}, 1H, H-C(3')); 6.92 (s, 1H, H-C(6)); 6.74-8.20 (m, 22H, ODmt, OBz, NPht).

### 1-{2'-O-[(Allyloxy)(diisopropylamino)phosphino]-3'O-benzoyl-4'-O-[(4,4'dimethoxytriphenyl)-methyl]- β-D-ribo-pyranosyl}-5-(2-phtalimidoethyl)- uracil

88 mg (0.11 mmol) 1-(3-O-Benzoyl-4-O-(4,4'-dimethoxytrityl)-(3-D-ribopyranosyl)-5-(2-phtalimidoethyl)uracil wurden in 5 ml Dichlormethan gelöst, mit 75 µl (0.44 mmol) N-Ethyl-diisopropylamin und 70 µl (0.3 mmol) Allyloxy-chlor-(diisopropylamino)phosphin versetzt und 3 h bei Raumtemperatur gerührt. Nach Zugabe von weiteren 35 µl (0.15 mmol) Allyloxy-chlor-(diisopropylamino)phosphin zur Vervollständigung der Reaktion wurde noch 1 h bei Raumtemperatur gerührt und das Reaktionsgemisch im Vakuum eingeengt. Chromatographie an Kieselgel (Ethylacetat/Heptan: Gradient 1:2 auf 1:1 auf 2: 1, jeweils mit 2% Triethylamin) lieferte 85 mg (76%) 1-{2'-O-[(Allyloxy)(diisopropylamino)phosphino]-3'O-benzoyl-4'-O-[(4,4'-dimethoxytriphenyl)-methyl]- β-D-ribo-pyranosyl}-5-(2-phtalimidoethyl)- uracil.
DC: R_{f} 0.36 (Ethylacetat/Heptan 2:1).
¹H-NMR (CDCl₃, 300 MHz): Ausgewählte charakteristische Lagen: 2.28, 2.52 (2 dd, J = 5.0, 11.0 Hz, 2 H, 2 H-5'), 3.79, 3.78 (app. 2 s, 12 H, OMe), 6.14 (1 bs, 1 H, H-3').
³¹P-NMR (CDCl₃): 149.8, 150.6

### 5.2 Indol-basierender Linker

N-Phthaloyltryptamin wird wie beschrieben aus Phthalsäureanhydrid und Tryptamin erhalten (Kuehne et al J. Org. Chem. **43,** 13, **1978,** 2733-2735). Dieses wird mit Boran-THF zum Indolin reduziert (analog A. Giannis, et al., Angew. Chem. 1989, 101, 220).

Das 3-substituierte Indolin wird zuerst mit Ribose zum Nucleosidtriol und dann mit Essigsäureanhydrid zum Triacetat umgesetzt. Man oxidiert mit 2,3-Dichlor-5,6-dicyanoparachinon und spaltet die Acetate mit Natriummethylat, benzoyliert selektiv in 2'-Position, DM-trityliert selektiv in 4'-Position, und führt die Wanderungsreaktion zum 3'-Benzoat durch. Die Bildung des Phosphoramidits erfolgt wie üblich. Dieser läßt sich ohne Änderung der Syntheseprotokolle für die automatisierte Oligonucleotidsynthese einsetzen.

### Durchführung

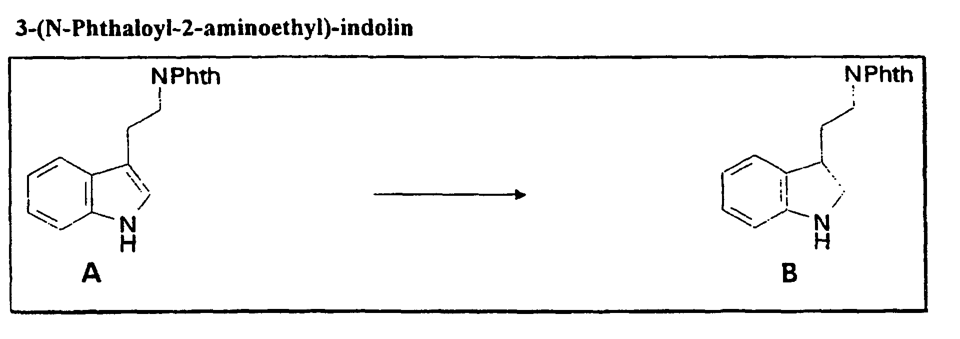

Unter Stickstoffatmosphäre wurden 51.4 g (177 mmol) Phthaloyltryptamin A in 354 ml 1M Boran-THF-Lösung (2 eq.) gelöst und auf 0 °C abgekühlt. Bei 0 °C wurde langsam 354 ml Trifluoressigsäure zugetropft (Vorsicht: Gasentwicklung) und 30 min. nachgerührt (DC-Kontrolle: EtOAc). Dann wurden 17.3 ml Wasser zugegeben, 10 min gerührt und im Vak. eingeengt. Der Rückstand wurde in 10%iger NaOH-Lösung / Dichlormethan gelöst, die organische Phase wurde abgetrennt, über NaSO₄ getrocknet, filtriert und im Vak. eingeengt. Der Rückstand (50.9 g) wurde aus heißem Ethanol (3 l) umkristallisiert. Man erhielt 41.4 g **B,** Smp. 161-162°C. Die Mutterlauge wurde im Vakuum eingeengt und der Rückstand erneut aus Ethanol umkristallisiert. Man erhielt weitere 3.2 g **B,** Smp. 158-159 °C.
Gesamtausbeute: 44.6 g (153 mmol) **B,** d. s. 86%.
¹H-NMR (CDCl₃, 300 MHz): 1.85-2.00, 2.14-2.28 (2 m, 2 x 1 H, CH₂CH₂NPhth), 2.70 (bs, 1 H, NH), 3.24-3.38, 3.66-3.86 (2 m, 5 H, CH₂CH₂NPhth, H-2a, H-2b, H-3), 6.62 (d, J = 8.0 Hz, 1 H, H-7), 6.66-6.72 (m, 1 H, H-5), 6.99 (app t, J = 7.5 Hz, 1 H, H-6), 7.14 (d, J = 8.0 Hz, 1 H, H-4), 7.64-7.74, 7.78-7.86 (2 m, 2 x 2 H, Phth).
¹³C-NMR (CDCl₃, 75 MHz): 32.70, 36.10 (2 t, C-2, CH₂CH₂NPhth), 39.62 (d, C-3), 53.04 (t, CH₂NPhth), 109.65 (d, C-7), 118.74 (d, C-5), 123.25 (d, Phth), 123.92, 127.72 (2 d, C-4, C-6), 131.81 (s, C-3a), 132.14 (s, Phth), 133.99 (d, Phth), 151.26 (s, C-7a), 168.38 (s, C=O).
Ber: C: 73.96, H: 5.52, N: 9.58; gef.: C: 73.89, H: 5.57, N: 9.55.
MS (ES⁺): 293 (MH⁺, 100%)

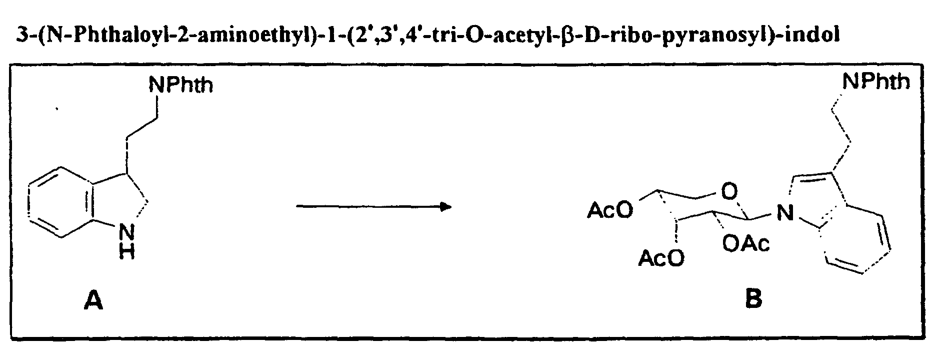

Unter Stickstoffatmosphäre wurden 45.2 g (155 mmol) A und 23.2 g (155 mmol; 1.0 eq.) D-Ribose in 750 ml trockenem Ethanol suspendiert und 4 h zum Rückfluß erhitzt (DC-Kontrolle: CH₂Cl₂/MeOH 10:1). Nach abkühlen auf RT wurde im Vak. eingeengt. Der Rückstand wurde in 300 ml Pyridin gelöst und unter Eiskühlung mit 155 ml Essigsäureanhydrid versetzt. Nach 15 min. wurde das Eisbad entfernt und 18 h bei RT gerührt (DC-Kontrolle: EtOAc/iso-Hexan 1:1). Diese Lösung wurde im Vakuum eingeengt und 3 mal mit je 300 ml Toluol coevaporiert. Das erhaltene Öl wird in 900 ml Dichlormethan gelöst und unter Eiskühlung mit 38.8 g (171 mmol; 1.1 eq.) 2,3-Dichlor-5,6-dicyanoparachinon versetzt. Nach 15 min. wurde das Eisbad entfernt und 1.5 h bei RT nachgerührt (DC-Kontrolle: EtOAc/iso-Hexan 1:1). Der ausgefallene Niedercshlag wurde abgesaugt und mit Dichlormethan gewaschen und verworfen. Das Filtrat wurde mit 600 ml ges. NaHCO₃-Lösung gewaschen. Der dabei ausgefallene Niederschlag wurde erneut abgesaugt und mit Dichlormethan gewaschen und verworfen. Die vereinigten organischen Extrakte wurden über NaSO₄ getrocknet und im Vak. eingeengt. Der Rückstand (90.9 g) wurde durch Flashchromatographie an Kieselgel 60 gereinigt (10 x 25 cm; EtOAc/iso-Hexan 2:3).
Man erhielt: 21.5 g reines **B** und 46.83 g Mischfraktionen, die nach erneuter Chromatographie weitere 20.4 g reines **B** lieferten.
Gesamtausbeute: 41.9 g (76 mmol) **B,** d. s. 49%.
¹H-NMR (CDCl₃, 300 MHz): 1.64, 1.98, 2.19 (3 s, 3 x 3 H, Ac), 3.06 (t, J = 8.0 Hz, 2 H, CH₂CH₂NPhth), 3.81-4.00 (m, 4 H, H-5'ax, H-5'eq, CH₂NPhth), 5.13 (ddd, J = 2.5, 6.0, 10.5 Hz, 1 H, H-4'), 5.36 (dd, J = 3.5, 9.5 Hz, 1 H, H-2'), 5.71 (d, J = 9.5 Hz, 1 H, H-1'), 5.74 (app t, J = 3.0 Hz, 1 H, H-3'), 7.02 (s, 1 H, H-2), 7.04-7.10, 7.13-7.19 (2 m, 2 x 1 H, H-5, H-6), 7,33 (d, J = 8.0 Hz, 1 H, H-7), 7.58-7.66, 7.72-7.80 (2 m, 5 H, Phth, H-4).
¹³C-NMR (CDCl₃, 75 MHz): 20.23, 20.65, 20.87 (3 q, Ac), 24.41, 38.28 (2 t, CH₂CH₂), 63.53 (t, C-5'), 66.24, 68.00, 68.64 (3 d, C-2', C-3', C-4'), 80.33 (d, C-1'), 109.79 (d, C-7), 113.95 (s, C-3), 119.33, 120.39, 122.04, 122.47 (4 d, C-4, C-5, C-6, C-7), 123.18 (d, Phth), 128.70, 132.17 (2 s, C-3a, Phth), 133.87 (d, Phth), 136.78 (s, C-7a), 168.24, 168.77, 169.44, 169.87 (4 s, C=O).
Ber: C: 63.50, H: 5.15, N: 5.11; gef.: C: 63.48, H: 5.16, N: 5.05.
MS (ES⁺): 566 (M+NH₄⁺, 82%), 549 (MH⁺, 74%), 114 (100%).

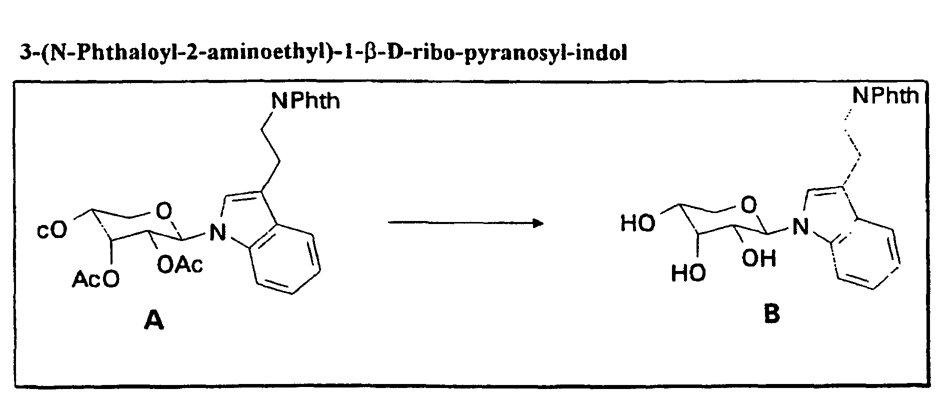

Unter Stickstoffatmosphäre wurden 44.1 g (80 mmol) A in 400 ml wasserfreiem Methanol gelöst. Unter Eiskühlung versetzte man mit 4.0 ml 30%iger Natriummethylatlösung und rührte dann 18 h bei RT. Der ausgefallene Niederschlag wurde abgesaugt und mit kaltem Ethanol gewaschen. Das Filtrat wurde im Vak. eingeengt. Der Rückstand wurde in Dichlormethan aufgenommen. Diese Lösung wurde mit ges. NaHCO₃-Lösung gewaschen, über NaSO₄ getrocknet und im Vak. eingeengt. Der erhaltene Rückstand wurde zusammen mit dem aus der Reaktionslösung ausgefallenen Niederschlag aus heißem Ethanol umkristallisiert. Man erhielt 22.6 g **B,** Smp. 196-198 °C. Die Mutterlauge wurde im Vakuum eingeengt und der Rückstand erneut aus Ethanol umkristallisiert. Man erhielt weitere 9.2 g **B,** Smp. 188-194 °C.
Gesamtausbeute: 25.8 g **B,** d. s. 76%.

¹H-NMR (MeOD, 300 MHz): 3.09 (app. t, J = 7.0 Hz, 2 H, CH₂CH₂NPhth), 3.64-3.98 (m, 5 H, H-4', H-5'ax, H-5'eq, CH₂NPhth), 4.05 (dd, J = 3.5, 9.5 Hz, 1 H, H-2'), 4.22 (app t, J = 3.0 Hz, 1 H, H-3'), 5.65 (d, J = 9.5 Hz, 1 H, H-1'), 6.95-7.05, 7.09-7.16 (2 m, 2 x 1 H, H-5, H-6), 7.25 (s, 1 H, H-2), 7.44 (d, J = 8.0 Hz, 1 H, H-7), 7.60 (d, J = 8.0 Hz, 1 H, H-4), 7.74-7.84 (m, 4 H, Phth).
¹³C-NMR (d₆-DMSO, 75 MHz): 23.87, 37.79 (2 t, CH₂CH₂NPhth), 64.82 (t, C-5'), 66.74 (d, C-4'), 68.41 (d, C-2'), 71.42 (d, C-3'), 81.37 (d, C-1'), 110.42 (d, C-7), 111.05 (s, C-3), 118.17, 119.21, 121.36, 122.92, 123.80 (5 d, C-2, C-4, C-5, C-6, NPhth), 127.86, 131.59 (2 s, C-3a, Phth), 134.27 (d, Phth), 136.62 (s, C-7a), 167.72 (s, C=O).
MS (ES⁻): 457 (M+OH⁻ +H₂O, 49%), 439 (M+OH⁻, 100%), 421 (M-H⁺, 28%)

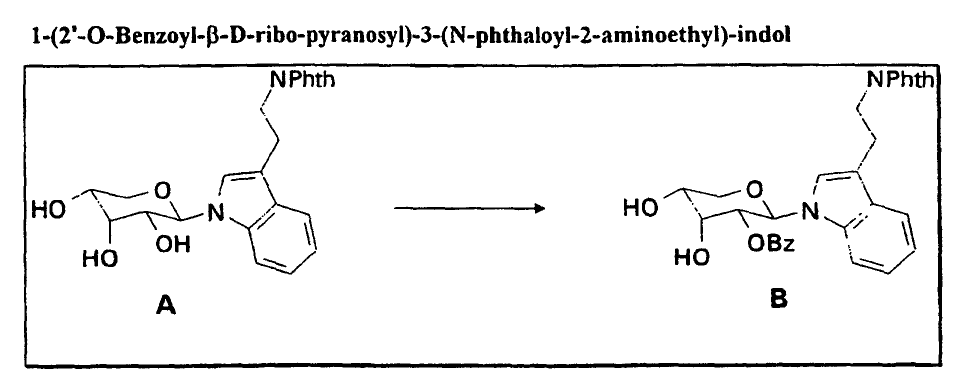

Unter Stickstoffatmoshäre wurde 10.6 g (25 mmol) A in 250 ml trockenem Dichlormethan aufgenommen. Man versetzt mit 305 mg DMAP (2.5 mmol) und 20 ml Pyridin. Es wurde erwärmt bis alles in Lösung war und anschließend auf -78 °C abgekühlt. Jetzt wurde 3.35 ml Benzoylchlorid (28.8 mmol) gelößt in 8 ml Dichlormethan innerhalb von 15 min zugetropft. DC-Kontrolle (EtOAc/Hexan 3:1) nach weiteren 30 min zeigte vollständige Reaktion an. Nach 45 min wurde die kalte Lösung über einen Faltenfilter direkt auf 200 ml ges. NH₄Cl-lösung gegeben und der Filterrückstand wurde mit Dichlormethan gewaschen. Die organische Phase wurde einmal mit Wasser gewaschen, über MgSO₄ getrochnet und eingeengt. Der Rückstand wurde 2 mal mit Toluol coevaporiert und durch Flashchromatographie an 10 x 20 cm Kieselgel mit EtOAc/Hexan 3:1 gereinigt. Man erhiehlt 8.1 g **B** (64%).
¹H-NMR (CDCl₃, 300 MHz): 2.45, 2.70 (2 bs, 2 x 1 H, OH), 3.04 (t, J = 8.0 Hz, 2 H, CH₂CH₂NPhth), 3.80-4.20 (m, 5 H, H-4', H-5'ax, H-5'eq, CH₂NPhth), 4.63 (bs, 1 H, H-3'), 5.46 (dd, J = 3.5, 9.5 Hz, 1 H, H-2'), 6.03 (d, J = 9.5 Hz, 1 H, H-1'), 7.08-7.31 (m, 5 H, H-2, H-5, H-6, Bz-m-H), 7.41-7.48 (m, 1 H, H-Bz-p-H), 7.50 (d, J = 8.0 Hz, 1 H, H-7), 7.64-7.79 (m, 7 H, Phth, H-4, Bz-o-H).
¹³C-NMR (d₆-DMSO, 75 MHz): 24.40, 38.22 (2 t, CH₂CH₂NPhth), 65.95 (t, C-5'), 66.65 (d, C-4'), 69.55 (d, C-3'), 71.87 (d, C-2'), 79.57 (d, C-1'), 109.96 (d, C-7), 113.70 (s, C-3), 119.21, 120.21, 122.11, 122.41, 123.14 (5 d, C-2, C-4, C-5, C-6, NPhth), 128.28 (d, Bz), 128.58, 128.59 (2 s, C-3a, Bz), 129.62 (d, Phth), 132.05 (s, Phth), 133.81 (Bz), 136.97 (s, C-7a), 165.12, 168.29 (2 s, C=O).
MS (ES⁻): 525 (M-H⁻, 12%), 421 (M-PhCO⁺, 23%), 107 (100%).

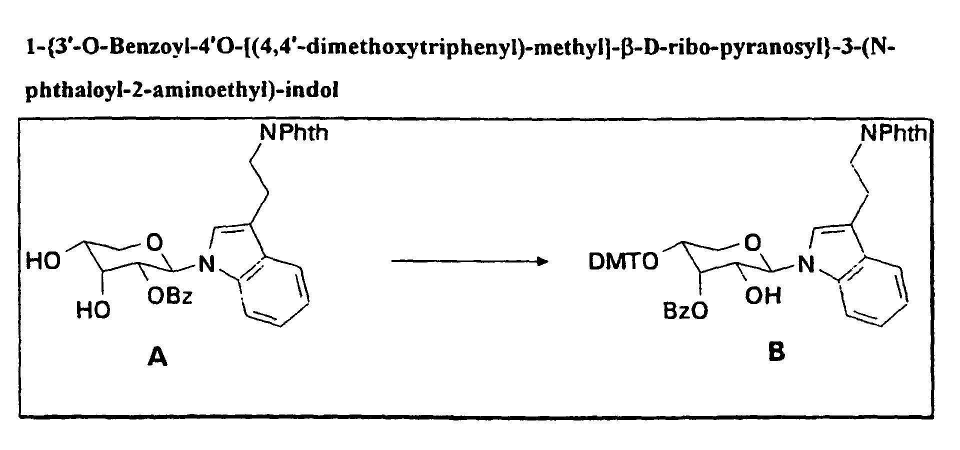

Unter Stickstoffatmoshäre wurde 8.9 g (16.9 mmol) A in 135 ml trockenem Dichlormethan suspendiert. Man versetzte mit 206 mg DMAP (1.68 mmol), 5.8 ml N-Ethyldiisopropylamin (33.7 mmol) und ca. 12 ml Pyridin (bis zur vollständigen Lösung). Jetzt wurde mit 34 g Molsieb 4Å versetzt und 30 min gerührt. Nach Abkühlen auf 0 °C wurde mit 11.4 g DMTCI (33.7 mmol) versetzt und nach Entfernen des Kühlbads 75 min gerührt. Dann wurden nochmals 1.94 g (0.34 eq) und nach weiteren 40 min 1.14 g (0.2 eq) und nach weiteren 65 min 1.14 g DMTCl (0.2 eq) zugegeben. Nach 4.25 h war die Reaktion beendet. Man versetzte dann mit 25.3 ml n-Propanol (20 eq), rührte noch 30 min nach und engte dann vorsichtig ein (Schaumbildung). Der Rückstand wurde in 100 ml Pyridin gelößt. Man versetzte mit 1.85 g DMAP (15.1 mmol; 0.9 eq), 13.05 ml N-Ethyldiisopropylamin (101 mmol; 6.0 eq), 71 ml n-Propanol (940 mmol; 56 eq) und 3.74 g p-Nitrophenol (26.9 mmol; 1.6 eq). Diese Mischung wurde unter Stickstoff 96 h bei 75-80 °C gerührt. Nach Abkühlen auf Raumtemperatur wurde die Mischung über Celite filtiert und eingeengt. Der Rückstand wurde durch Flashchromatographie an 9 x 17 cm Kieselgel mit Toluol/Diethylether/Triethylamin 90:10:1 gereinigt. Die produkthaltuigen Fraktionen (9.25 g) wurden zunächst aus EtOAc umkristallisiert und anschließend aus Toluol/Methanol umgefällt. Man erhielt 5.86 g **B** (42%).

¹H-NMR (CDCl₃, 300 MHz): 2.64 (bs, 1 H, OH), 2.68 (dd, J = 5.0, 11.5 Hz, 1 H, H-5'eq), 2.94 (dd, J = 7.5, 16.0 Hz, 1 H, CH₂CH₂NPhth), 3.03 (dd, J = 8.0, 16.0 Hz, 1 H, CH₂CH₂NPhth), 3.67-3.74 (m, 1 H, H-5'ax), 3.69, 3.70 (2 s, 2 x 3 H, OMe), 3.85 (t, J = 7.5 Hz, 2 H, CH₂CH₂NPhth), 3.94 (ddd, J = 3.0, 5.0, 10.5 Hz, 1 H, H-4'), 4.03 (dd, J = 3.5, 9.0 Hz, 1 H, H-2'), 5.51 (d, J = 9.0 Hz, 1 H, H-1'), 5.86 (bs, 1 H, H-3'), 6.68-7.66 (m, 25 H), 8.19-8.30 (m, 2 H).
¹³C-NMR (CDCl₃, 75 MHz): 24.16, 38.80 (2 t, CH₂CH₂NPhth), 55.25, 55.26 (2 q, Ome), 65.58 (t, C-5'), 68.29, 69.19, 73,83 (3 d, C-2', C-3', C-4'), 83.03 (d, C-1'), 87.31 (CAr₃)110.03 (d, C-7), 113.37, 113.47 (2 d), 113.53 (s, C-3), 118.95, 120.20, 122.28, 122.31, 123.10, 127.07, 128.02, 128.08, 128.68 (9 d), 128.74 (s), 130.02, 130.19, 130.22 (3 d), 130.37, 131.95 (2 s), 133.40, 133.83 (2 d), 135.98, 136.14, 136.56, 145.12, 158.82, 166.76, 168.52 (7 s, C-7a, 2 COMe, 2 C=O).

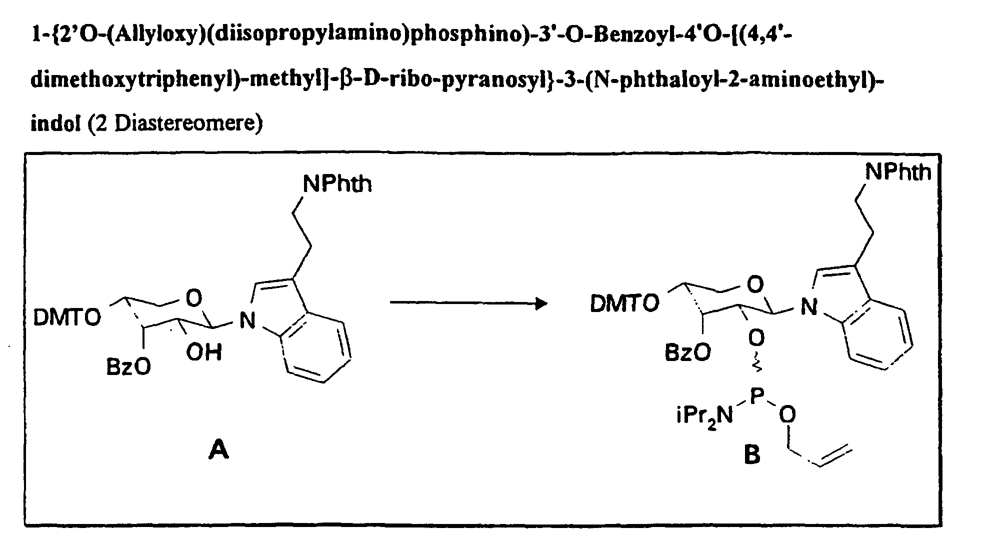

Unter Argonatmosphäre wurde 1658 mg Alkohol A (2.0 mmol) in 10 ml trockenem Dichlormethan gelößt. Man versetzte mit 1.03 ml N-Ethyldiisopropylamin (6.0 mmol) und 0.63 ml Phosphorigsäuremonoallylesterdiisopropylamidchlorid (2.8 mmol) und rührte 1 h bei Raumtemperatur. Dann wurde das überschüssige Phosphorylierungsreagenz durch Zugabe von 61 µl (0.8 mmol) Isopropanol zerstört. Nach 10 min wurde im Vakuum eingeengt und der Rückstand durch Flashchromatographie an 3.3 x 21 cm Kieselgel mit Hexan/EtOAc/NEt₃ (75:25:1) gereinigt. Die produkthaltigen Fraktionen wurden eingeengt, in CCl₄ aufgenommen und wieder eingeengt. Man erhielt 2.04 g eines fast farblosen Schaums (quant.), der so direkt zur Oligomerisierung verwendet werden kann und bei -20 °C mehrere Wochen haltbar ist.
DC auf Kieselgel (EtOAc/Hexan/ NEt₃ 33:66:1): 0.41
¹H-NMR (CDCl₃, 300 MHz): Ausgewählte charakteristische Lagen: 2.42, 2.53 (2 dd, J = 5.0, 11.0 Hz, 2 H, 2 H-5'eq), 3.76, 3.77, 3.78, 3.79 (4 s, 4 x 3 H, OMe), 5.70, 5.73 (2 d, J = 9.0 Hz, 2 H, 2 H-1'), 6.16, 6.29 (2 bs, 2 H, 2 H-3').
³¹P-NMR (CDCl₃): 150.6, 151.0

### 5.3 Lysin-basierender Linker

Die Synthese ist im Schema 7 abgebildet und wird im folgenden im Detail beschrieben.

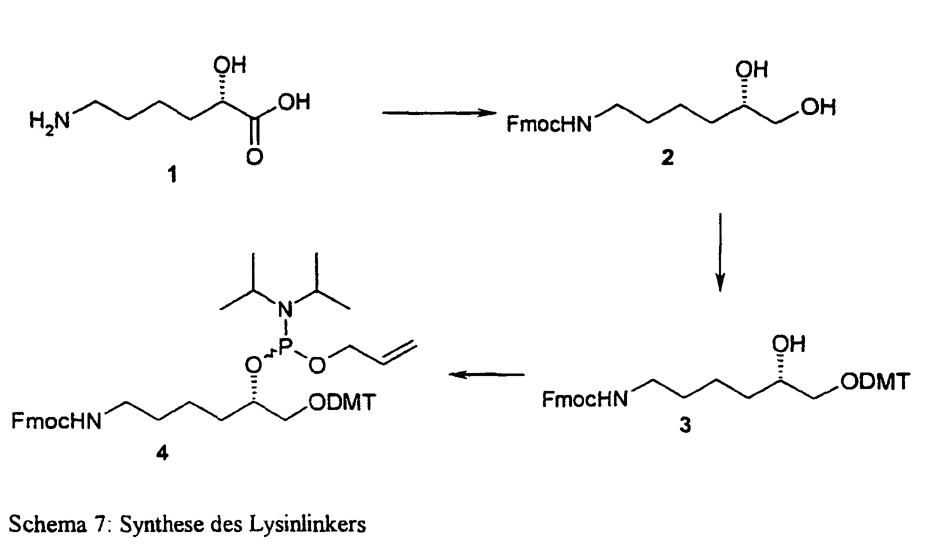

6-Amino-2(S)-hydroxyhexansäure **(1)** wurde nach literaturbekannter Weise durch Diazotierung und anschließebnde Hydrolyse aus L-Lysin hergestellt (K.-I. Aketa, Chem. Pharm Bull. 1976, 24, 621).

### 2-(S)-N-Fmoc-6-amino-1,2-hexandiol (2)

3.4 g LiBH₄ (156 mmol, 4 eq) werden unter Argon in 100 ml abs. THF gelöst (exotherm!). Nach Abkühlen auf ca. 30 °C werden 39.6 ml TMSCI (312 mmol, 8 eq) langsam zugetropft (Gasentwicklung!), wobei sich ein Niederschlag bildet. Anschließend werden im Argon-Gegenstrom 5.74 g 6-Amino-2(S)-hydroxyhexansäure (**1**) (39 mmol) portionsweise zugegeben und es wird auf 65 °C erwärmt, bis das DC (Kieselgel; i-PrOHlkona. NH₄OH/Wasser 7:2:1; Anfärben mit Ninhydrin) kein Edukt mehr zeigt (ca. 3 h). Unter Eiskühlung wird vorsichtig mit 120 ml Methanol versetzt (starke Gasentwicklung!). Das Lösungsmittel wird im Vakuum eingeengt, der Rückstand wird 3 mal mit je 200 ml Methanol coevaporiert und anschließend in 100 ml abs. DMF gelöst. Nach Zugabe von 16 ml Ethyldiisopropylamin (93.6 mmol, 2.4 eq) wird die Mischung auf 0°C gekühlt und portionsweise mit 12.1 g FmocCl (46.8 mmol, 1.2 eq) versetzt. Nach 15 Minuten wird das Kühlbad entfernt und bei Raumtemperatur gerührt bis das Edukt verbraucht ist (ca. 3 h; DC-Kontrolle: Kieselgel; CHCl₃/MeOH/HOAc/Wasser 60:30:3:5). Die Reaktionslösung wird auf 600 ml ges. NaHCO₃-Lösung gegeben. Der Niederschlag wird abfiltriert, mit 200 ml Wasser gewaschen und bei 50 °C am HV getrocknet bis zur Gewichtskonstanz (ca. 6 h). Man erhält 13.9 g eines farblosen Feststoffs, der aus Ethylacetat (40 ml)/n-Hexan (35 ml) umkristallisiert wird. Ausbeute: 9.05 g (65%).

¹H-NMR (300 MHz, CDCl₃): 7.68, 7.51 (2 d, J = 8.0 Hz, je 2 H, Ar-H), 7.32 (t, J = 8.0 Hz, 2 H, Ar-H), 7.23 (dt, J = 1.6, 8.0 Hz, 2 H, Ar-H), 4.92 (bs, 1 H. NH), 4.32 (d, J = 7.0 Hz, 2 H, OCOCH₂), 4.13 (bt, J = 7.0 Hz, 1 H, OCOCH₂CH), 3.64-3.58 (m, 1 H, H-1, H-1', H-2, H-6, H-6'), 3.54 (dd, J = 3.2, 11.0 Hz, 1 H, H-1, H-1', H-2, H-6, H-6'), 3.35 (dd, J = 7.4, 11.0 Hz, 1 H, H-1, H-1', H-2, H-6, H-6'), 3.16-3.06 (m, 2 H, H-1, H-1', H-2, H-6, H-6'), 3.0-2.0 (bs, 2 H, OH), 1.52-1.18 (m, 6 H, H-3, H-3', H-4, H-4', H-5, H-5').

2-(S)-N-Fmoc-O¹-DMT-6-amino-1,2-hexandiol (**3**) wurde nach WO 89/02439 DM-trityliert.

### 2-(S)-N-Fmoc-O¹-DMT-O²-allyloxydiisopropylaminophosphinyl-6-amino-1,2-hexandiol (4)

Zu einer Lösung von 670 mg des Alkohols (**3**) (1.02 mmol) in 10 ml abs. Dichlormethan werden unter Argon 0.51 ml Ethyldiisopropylamin (3.0 mmol, 3 eq) und 0.33 ml Chlor-N,N-diisopropylaminoallyloxyphosphin ( 1.5 mmol, 1.5 eq) gegeben. Man rührt 2 h bei Raumtemperatur, zieht das Lösungsmittel im Vakuum ab und reinigt den erhaltenen Rückstand durch Flashchromatographie an 3.2 x 16 cm Kieselgel (EtOAc/iso-Hexan/NEt₃ 20:80:1). Man erhält 839 mg (97%) eines leicht gelblichen Öls.

DC: Kieselgel; EtOAc/iso-Hexan/NEt₃ 50:50:1; UV; R_{f} = 0.77.

¹H-NMR (300 MHz, CDCl₃): 7.70-6.68 (m, 21 H, Ar-H), 4.92-4.62 (m, 1 H. NH), 4.31 (d, J = 7.0 Hz, 2 H, OCOCH₂), 4.13 (t, J = 7.0 Hz, 1 H, OCOCH₂CH), 3.98-3.40 (m, 5 H), 3.77 (2 s, je 3 H, OMe), 3.16-2.86 (m, 4 H), 2.58 (t, J = 7.0 Hz, 1 H, CHCN), 2.38 (t, 1 H, CHCN), 1.80-1.20 (m, 6 H), 1.20, 1,18, 1.17, 1.16, 1.15, 1.13, 1.08, 1.06 (8 s, 12 H, NMe).

³¹P-NMR (300 MHz, CDCl₃): 149.5, 149.0 (2 s)

### Beispiel 6

### Synthese eines p-RNA-Oligos der Sequenz 4'-Indollinker-A8-2' unter Verwendung von Benzimidazoliumtriflat als Kupplungsreagenz

108 mg Indollinker-Phosphoramidit und 244 mg A-Phosphoramidit werden in Synthesizergläschen eingewogen und für 3 h im Exsikkator über KOH zusammen mit dem mit 28,1 mg CPG-Träger, beladen mit A-Baustein, gefüllten Säulchen am HV belassen. Die Phosphoramidite werden in 1 ml (Indollinker) bzw. 2,5 ml (A-Phosphoramidit) Acetonitril gelöst und einige Kügelchen von dem Molsieb zugesetzt und über KOH geschlossen im Exsikkator belassen.
200 mg Jod werden unter starkem Rühren in 50 ml Acetonitril gelöst. Nachdem alles gelöst ist (Sichtkontrolle) werden 23 ml Wasser und 4,6 ml sym-Collidin zugegeben und die Lösung einmal gut durchmischt. Zum Detritylieren wird eine 6 %ige Lösung von Dichloressigsäure in Dichlormethan eingesetzt. Das Cappinereagenz (Acetanhydrid + Base) wird wie für Oligonucleotidsynthese üblich gekauft und verwendet.
Benzimidazoliumtriflat wird aus heißem Acetonitril umkristallisiert und getrocknet. Mit den fast farblosen Kristallen wird eine 0,1 M Lösung in wasserfreiem Acetonitril als Kupplungsreagenz hergestellt. Während der Synthese bleibt diese Lösung immer klar und es kommt zu keinen Verstopfungen der Synthesizerschläuche.
Abgeänderter DNA-Kupplungscyclus am Eppendorf Ecosyn 300+ (DMT-on):

| Detritylierung | 7 Minuten |
|---|---|
| Kupplung | 1 Stunde |
| Capping | 1,5 Minuten |
| Oxidation | 1 Minute |

20 mg Tetrakis(triphenylphosphin)palladium wird in 1,5 ml Dichlormethan gelöst, 20 mg Diethylammoniumhydrogencarbonat, 20 mg Triphenylphosphin und der das Oligonucleotid tragende Glasträger zugesetzt, dicht verschlossen (Parafilm) und das Gläschen 5 h bei RT. bewegt. Dann wird der Glasträger über eine Analysennutsche abgesaugt, und mit Dichlormethan, mit Aceton und mit Wasser gewaschen.

Der Träger wird mit wäßriger 0,1 molarer Natriumdiethyldithiocarbamatlösung aufgeschlämmt und 45 min bei RT. belassen. Man saugt ab, wäscht mit Wasser, Aceton, Ethanol und Dichlormethan. Der Träger wird in 1,5 ml 24 %iger Hydrazinhydratlösung suspendiert, 24-36 h lang bei 4 °C gerüttelt und mit 0,1molarem Triethylammoniumhydrogencarbonatpuffer (TEAB-Puffer) auf 7 ml verdünnt. Über eine Waters Sep-Pak Cartridge wurde hydrazinfrei gewaschen. Es wird mit 5 ml einer 80%igen Ameisensäurelösung versetzt, und nach 30 min zur Trockene einrotiert. Der Rückstand wird in 10 ml Wasser aufgenommen, mit Dichlormethan extrahiert, die wäßrige Phase eingeengt und nun HPL-chromatographiert (tR = 33 min, Gradient von Acetonitril in 0,1M Triethylammoniumacetat-Puffer). Übliche Entsalzung (Waters Sep-Pak Cartridge) liefert das Oligonucleotid.
Ausbeute: 17, 6 OD.
Substanzidentität nachgewiesen durch ESI-Massenspektroskopie:
M(ber) = 3082 D, (M+2H)²⁺(gef) = 1541,9 D.

### Beispiel 7

### Herstellung von Konjugaten

### 1. Sequentielles Verfahren

Zuerst wird, wie in Beispiel 2 beschrieben, ein p-RNA-Oligomeres der Sequenz A₈, d. h. ein Octamer, auf dem Eppendorf Ecosyn D 300+ hergestellt und dann die folgenden Reagenzien ausgetauscht: 6 %ige Dichloressigsäure gegen 2 %ige Trichloressigsäure, Jod in Collidin gegen Jod in Pyridin, Benzimidazoliumtriflatlösung gegen Tetrazollösung. Nach Änderung des Syntheseprogramms wird ein DNA-Oligomeres der Sequenz GATTC nach bekannten Methoden (M. J. Gait, Oligonucleotide Synthesis, IRL Press, Oxford, UK 1984) weiter synthetisiert. Die Deallylierung, Hydrazinolyse, HPL-Chromatographie und Entsalzung erfolgt wie für das p-RNA-Oligomere beschrieben (siehe oben) und liefert das gewünschte Konjugat.

### 2. Konvergentes Verfahren

Wie in Beispiel 2 beschrieben, wird ein p-RNA-Oligomeres mit der Sequenz 4'-Indollinker-A₈-2' hergestellt, aufgereinigt, und jodacetyliert. Ein DNA-Oligomeres der Sequenz GATTC-Thiol-Linker wird nach bekannten Methoden (M. J. Gait, Oligonucleotide Synthesis, IRL Press, Oxford, UK 1984) synthetisiert und aufgereinigt (3'-Thiol-Linker von Glen Research: Nr. 20-2933). Beim Stehenlassen der beiden Fragmente (T. Zhu et al., Bioconjug. Chem. 1994, 5, 312) in gepufferter Lösung entsteht das Konjugat, das abschließend über HPLC aufgereinigt wird.

### Beispiel 8

### Konjugation eines Biotinrestes an eine aminomodifizierte p-RNA:

Zuert wurde analog wie in Beispiel 6 beschrieben, ein p-RNA Oligomer der Sequenz TAGGCAAT, welches am 4'-Ende mittels des 5'-Aminomodiner 5 von Eurogentec (2-(2-(4-Monomethoxytrityl)aminoethoxy)ethyl-(2-cyanoethyl)-(N,N-diisopropyl)-phosphoramidit) mit einer Aminogruppe versehen ist, synthetisiert und aufgearbeitet. Das Oligonucleotid (17,4 OD, 0,175 µmol) wurde in 0,5 ml basischem Puffer aufgenommen, 1,14 mg (2,5 µmol) Biotin-N-hydroxysuccinimidester in 114 µl DMF (abs.) gelöst und 1h bei RT stehengelassen. Das entstandene Konjugat wurde mittels präparativer HPLC gereinigt und das reine Produkt mit einer Sepak entsalzt.

Ausbeute: 8.6 OD (49%)
M (ber.) = 3080 D, M (fef) = 3080,4 D

### Beispie) 9

### Herstellung Cyanin- bzw. Biotirr-markierter p-RNA online

Die verschiedenen A,T,G,C und Ind (Ind = Aminoethylindol als Nucleobase) Phosphoramidite wurden zuerst nach bekannten Verfahren hergestellt. Cyanin (Cy3-CE) und Biotin Phosphoramidite wurden bei Glen Research erhalten.

Die vollautomatische Festphasensynthese wurde mit jeweils 15 µmol durchgeführt. Ein Synthesezyklus besteht aus den folgenden Schritten
(a) Detritylierung: 5 Minuten mit 6% DCA (Dichloressigsäure) in CH₂Cl₂ (79 ml);
(b) Waschen mit CH₂Cl₂ (20 ml), Acetonitril (20 ml) und danach spülen mit Argon;
(c) Kupplung: Waschen des Harz mit dem Aktivator (0,5 M Pyridin.HCl in CH₂Cl₂, 0,2 ml;
   30-minütiges Behandeln mit Aktivator (0,76 ml) und entsprechendes Phosphoramidit (0,76 ml : 8 eq; 0,1 M in Acetonitril) im Verhältnis 1:1;
(d) Capping: 2 Minuten mit 50% Cap A (10,5 ml) und 50% Cap B (10,5 ml) von Perseptive (Cap A: THF, Lutidine, Acetanhydrid; Cap B: 1-Methylimidazol, THF, Pyridin).
(e) Oxidation: 1 Minute mit 120 ml Iodlösung (400 mg Jod in 100 ml Acetonitril, 46 ml H₂O und 9,2 ml sym-Collidine).
(f) Waschen mit Acetonitril (22 ml).

Zur Erleichterung der nachfolgenden HPLC-Reinigung der Oligonucleotide wurde die letzte DMT(Dimethoxytrityl)- bzw. MMT (Monomethoxytrityl)-Schutzgruppe von Biotin- bzw. Cyanin-Monomeren nicht abgespalten. Der Nachweis der letzten Kupplung mit den modifizierten Phorphoramiditen erfolgt nach der Synthese mit 1% des Harzes mittels Tritylkationabsorption in UV (503 nm).

### Aufarbeitung des Oligonucleotids:

Die Abspaltung der Allyletherschutzgruppen erfolgte mit einer Lösung von Tetrakis(triphenylphosphin)palladium (272mg), Triphenylphosphin (272 mg) und Diethylammoniumhydrogencarbonat in CH₂Cl₂ (15ml) nach 5 Stunden bei RT. Die Glasträger werden danach mit CH₂CL₂ (30ml), Aceton (30ml) und Wasser (30ml) gewaschen. Um Palladiumkomplexreste zu entfernen, wurde das Harz mit einer wäßrigen 0,1 M Natriumdiethyldithiocarbamathydratlösung gespült. Die oben erwähnte Waschoperation wurde in einer umgekehrten Reihe noch einmal durchgeführt. Anschließend wurde das Harz am Hochvakuum 10 Minuten getrocknet. Der Abspaltungsschritt vom Glasträger bei gleichzeitiger Debenzoylierung wurde in 24% Hydrazinhydratlösung (6ml) bei 4°C durchgeführt. Nach HPLC-Kontrolle an RP 18 (18-25 Stunden) wurde das Oligonucleotid "Trityl ON" mittels einer aktiviert (Acetonitril, 20 ml) Waters Sep-Pak Cartridge vom Hydrazin befreit. Das Hydrazin wurde mit TEAB 0,1M (30ml) gewaschen. Das Oligonucleotid wurde dann mit Acetorutril/TEAB 0,1M (10ml) eluiert. Man reinigte mittels HPLC (zur Abtrennung von Abbruchsequenzen) und führte die DMT-Entschützung (30 ml 80%ig wäßrige Ameisensäure) durch. Abschließende Entsalzung (über Sep-Pak Kartuche, mit TEAB Puffer 0,1M/Acetonitril: 1/1) lieferte die reinen Cyanin- bzw. Biotinmarkierten Oligomeren.

Ein Aliquot dieser Oligolösung wurde für die Durchführung einer ESI-MS verwendet.
4' Cy-AIndTTCCTA 2': berechnet M = 3026,
gefunden (M+H)⁺ = 3027.
4' Biotin-TAGGAAIndT 2': berechnet M = 3014,
gefunden (M+2H)²⁺ m/z 1508 und (m+H)⁺ m/z 3015.

Die Oligos wurden zur Lagerung gefriergetrocknet.

### Beispiel 10

### Jodacetylierung von p-RNA mit N-(jodacetyloxy)-succinimid

### p-RNA-Sequenz : 4' AGGCAIndT 2' M_{w} = 2266,56 g/mol (Ind = Indol-CH₂-CH₂-NH₂-Linker)

1 eq. der *p*-RNA wurde in einer 0,1 molaren Natriumhydrogencarbonatlösung (pH 8,4) gelöst (1 ml pro 350 nmol) und mit einer Lösung von N-(jodacetyloxy)-succinimid in DMSO versetzt (40 µl pro mg). Man dunkelte den Ansatz mit Aluminiumfolie ab und beließ ihn bei Raumtemperatur für 30-90 Minuten.

Der Fortgang der Reaktion wurde mittels analytischer HPLC verfolgt. Die Standardbedingungen sind :
Puffer A : 0,1 molarer Triethylammoniumacetat-Puffer in Wasser
Puffer B : 0,1 molarer Triethylammoniumacetat-Puffer in Wasser:Acetonitril 1:4
Gradient: von 10% B startend auf 50% B in 40 Minuten
Säulenmaterial : 10 µM LiChrosphere ® 100 RP-18 von Merck Darmstadt GmbH; 250 x 4 mm
Retentionszeit der Edukte: 18,4 Minuten
Retentionszeit der Produkte in diesem Falle : 23,1 Minuten

Nach beendeter Reaktion wurde der Ansatz mit Wasser auf das vierfache Volumen verdünnt. Man aktivierte eine Waters Sep-Pak-Kartusche RP-18 (ab 15 OD 2 g Füllung) mit 2 x 10 ml Acetonitril und 2 x 10 ml Wasser, trug das Oligo auf, ließ es einsinken, wusch das Reaktionsgefäß mit 2 x 10 ml Wasser, wusch mit 3 x 10 ml Wasser nach, um Salz und Reagenz zu entfernen, und eluierte zuerst mit 5 x 1 ml 50:1 Wasser : Acetonitril und anschließend mit 1:1. Das Produkt eluierte in den 1:1-Fraktionen in sehr guter Reinheit. Die Fraktionen wurden in der Kälte und im Dunkeln eingeengt, vereinigt, und wieder eingeengt.

Die Ausbeuten wurden mittels UV-Absorptionspektrometrie bei 260 nm bestimmt.

### Massenspektromethe :

- Sequenz :: 4' AGGCAInd(CH2CH2NHCOCH2-I)T 2'
- berechnete Masse :: 2434,50 g/mol
- gefundene Masse MH₂²⁺:: 1217,9 g/mol = 2433 g/mol

### Beispiel 11

### Konjugation von p-RNA an ein Peptid der Sequenz CYSKVG:

Die jodacetylierte *p*-RNA (M_{w} = 2434,50 g/mol) wurde in einem Puffersystem gelöst (1000µl pro 114 nmol) und dann mit einer Lösung des Peptides in Puffer versetzt (2 mol CYSKVG-Peptid; M_{w} = 655, 773 g/mol; 228 nmol in 20 µl Puffer).

Wasser : Acetonitril 1:1. Die Produkt-Fraktionen wurden eingeengt, vereinigt und wieder eingeengt.
Die Ausbeuten wurden mittels UV-Absorptionspektrometrie bei 260 nm bestimmt. Sie erreichten 70-95% der Theorie.

### Massenspektrometrie :

- Sequenz :: 4' AGGCAInd(CH2CH2NHCOCH2-CYSKVG)T 2'
- berechnete Masse :: 2962,36 g/mol
- gefundene Masse MH₂²⁺:: 1482,0 g/mol = 2962 g/mol

### Beispiel 12

### Konjugation von p-RNA an eine Peptidbibliothek

Die jodacetylierte *p*-RNA (M_{w} = 2434,50 g/mol wurde in einem Puffersystem gelöst (1300 µl pro 832 nmol) und dann mit einer Lösung der Peptidbibliothek (CKR-XX-OH); X = Arg, Asn, Glu, His, Leu, Lys, Phe, Ser, Trp, Tyr) in Puffer versetzt (8 mol; mittlere Molekülmasse Mₘ = 677,82 g/mol; 4,5 mg = 6,66 µmol in 200 µmol Puffer).

Puffersystem: Borax/HCl-Puffer der Firma Riedel-de Haen, pH 8,0, wurde im Verhältnis 1:1 mit einer 10 millimolaren Lösung von EDTA-Dinatriumsala in Wasser gemischt und auf pH 6,6 mit HCl eingestellt. Man erhielt dadurch eine Lösung, die 5 mM Na₂EDTA enthält.

Man beließ den Ansatz bis zum vollständien Umsatz bei Raumtermperatur im Dunkeln. Die Reaktion wurde mittels HPLC-Analytik verfolgt. In diesem Fall war das Edukt nach 70 Stunden verschwunden.

Die Standardbedingungen der analytischen HPLC sind:
Puffer A : 0,1 molarer Triethylammoniumacetat-Puffer in Wasser
Puffer B : 0,1 molarer Triethylammoniumacetat-Puffer in Wasser:Acetonitril 1:4
Gradient: von 10% B startend auf 50% B in 40 Minuten
Säulenmaterial : 10 µM LiChrosphere® 100 RP-18 von Merck Darmstadt GmbH; 250 x 4
Retentionszeit des Eduktes : 18,8 Minuten
Retentionszeit des Produktes: mehrere Peaks von 13,9-36,2 Minuten

Nach beendeter Reaktion wurde der Ansatz mit Wasser auf das vierfache Volumen verdünnt. Man aktivierte eine Waters Sep-Pak-Kartusche RP-18 (ab 15 OD 2g Füllung) mit 3 x 10 ml Acetoniril und 3 x 10 ml Wasser, trug das Oligo auf, ließ es einsinken, wusch das Reaktionsgefäß mit 2 x 10 ml Wasser nach, wusch die Kartusche mit 3 x 10 ml Wasser nach, um Salz und überschüssiges Peptid zu entfernen, und eluierte mit 1:1 Wasser : Acetonitril, bis UV-spektroskopisch kein Prodikt mehr eluierte. Die Franktionen wurden in Kälte und im Dunkeln eingeengt, vereinigt und wieder eingeengt.

## Patentansprüche

1. Konjugat, enthaltend ein Pentopyranosyl-Nucleosid oder eine Pentopyranosyl-Nukleinsäure und ein Biomolekül ausgewählt aus der Gruppe der Peptide, Proteine oder Nucleinsäuren.

2. Konjugat nach Anspruch 1, enthaltend ein Pentopyranosyl-Nucleosid der Formel (I), 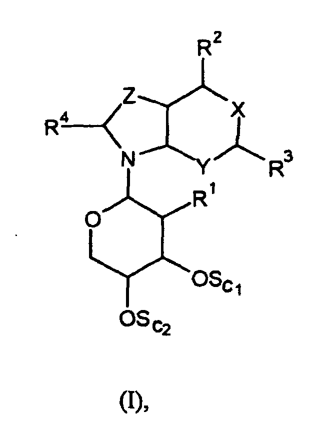 worin
R¹ gleich H, OH. Hal mit Hal gleich Br oder Cl oder oder ein Rest ausgewählt aus 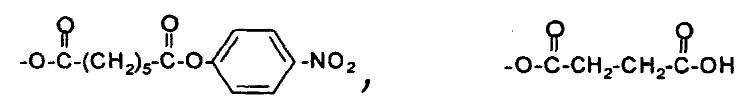 oder
- O-P[N(i-Pr)₂](OCH₂CH₂CN) mit i-Pr gleich Isopropyl, oder OS_{c3} mit S_{c3} gleich eine Schutzgruppe, vorzugsweise eine basen- oder metallkatalysiert abspaltbare Schutzgruppe, insbesondere eine Acylgruppe, besonders bevorzugt eine Benzoylgruppe,
R², R³ und R⁴ unabhängig voneinander, gleich oder verschieden, jeweils H, Hal mit Hal gleich Br oder Cl, NR⁵R⁶, OR⁷, SR⁸, =O, CₙH₂ₙ₊₁ mit n eine ganze Zahl von 1-12, vorzugsweise 1-8, insbesondere 1-4, eine β-eliminierbare Gruppe, vorzugsweise eine Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR¹⁰R¹¹ mit R¹⁰R¹¹ gleich H, CₙH₂ₙ₊₁ oder R¹⁰R¹¹ verbunden über einen Rest der Formel 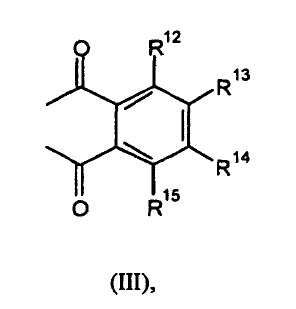 worin R¹², R¹³, R¹⁴ und R¹⁵ unabhängig voneinander, gleich oder verschieden, jeweils H, OR⁷, wobei R⁷ die oben genannte Bedeutung hat, oder CₙH₂ₙ₊₁, oder CₙH₂ₙ₋₁,wobei n die oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)-oder -N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet, und
S_{c1} und S_{c2} unabhängig voneinander, gleich oder verschieden, jeweils H oder eine Schutzgruppe ausgewählt aus einer Acyl-, Trityl- oder Allyloxycarbonylgruppe, vorzugsweise eine Benzoyl- oder 4, 4'-Dimethoxytrityl-(DMT-)gruppe,
oder der Formel (II) 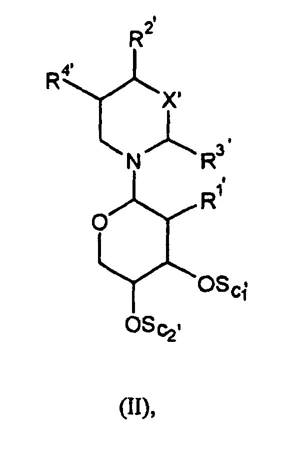 worin R^{1'} gleich H, OH,Hal mit Hal gleich Br oder Cl, oder ein Rest ausgewählt aus 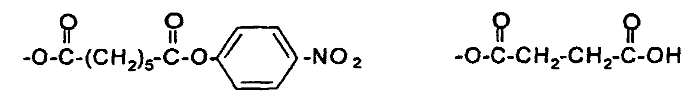 oder
- O-P[N(i-Pr)₂] (OCH₂CH₂CN) mit i-Pr gleich Isopropyl, oder OS_{c3} mit S_{c3} gleich eine Schutzgruppe, vorzugsweise eine basen- oder metallkatalysiert abspaltbare Schutzgruppe, insbesondere eine Acylgruppe, besonders bevorzugt eine Benzoylgruppe,
R^{2'},R^{3'} und R^{4'} unabhängig voneinander, gleich oder verschieden, jeweils H, Hal mit Hal gleich Br oder Cl, =O, CₙH₂ₙ₊₁ oder OCₙH₂ₙ₋₁,eine β-eliminierbare Gruppe, vorzugsweise eine Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR^{10'}R^{11'}, wobei R^{10'}, R^{11'}, unabhängig voneinander die oben genannte Bedeutung von R¹⁰ bzw. R¹¹ hat, und
X' =N-, =C(R^{16'})- oder -N(R^{17'})- bedeutet, wobei R^{16'} und R^{17'} unabhängig voneinander die oben genannte Bedeutung von R¹⁶ bzw. R¹⁷ haben, und S_{c1'} bzw. S_{c2'} die oben genannte Bedeutung von S_{c1} bzw. S_{c2} haben,

3. Konjugat nach Anspruch loder 2, **dadurch gekennzeichnet, daß** ein Allyloxy-Linker der Formel
S_{c4}NH(CₙH₂ₙ)CH(OPS_{c5}S_{c6})CₙH₂ₙOS_{c7} (IV),
worin S_{c4} und S_{c7} unabhängig voneinander, gleich oder verschieden, jeweils eine Schutzgruppe insbesondere ausgewählt aus Fmoc und DMT,
S_{c5} und S_{c6} unabhängig voneinander, gleich oder verschieden, jeweils eine Allyloxyund/oder Diisopropylamino-Gruppe und n eine ganze Zahl von 1 bis 12 sein kann, bedeuten,
eingebaut ist.

4. Konjugat nach einem der Ansprüche 1-3, **dadurch gekennzeichnet dass** das Pentopyranosyl-Nucleosid ein Ribo-, Arabino-, Lyxo- und/oder Xylo-pyranosyl-Nucleosid, vorzugsweise ein Ribopyranosyl-Nucleosid, ist.

5. Konjugat nach einem der Ansprüche 1-4, **dadurch gekennzeichnet, daß** der Pentopyranosyl-Teil des Pentopyranosyl-Nucleosids D- oder L-konfiguriert ist.

6. Konjugat nach einem der Ansprüche 1-5, **dadurch gekennzeichnet, daß** als Pentopyranosyl-Nucleosid ein Pentopyranosyl-purin, -2,6-diaminopurin, -6-purinthiol, - pyridin, -pyrimidin, -adenosin, -guanosin, -isoguanosin, -6-thioguanosin, -xanthin, - hypoxanthin, -thymidin, -cytosin, -isocytosin , -indol, -tryptamin, -N-phthaloyltryptamin, - uracil, -coffein, -theobromin, -theophyllin, -benzotriazol oder -acridin, eingesetzt wird.

7. Konjugat nach einem der Ansprüche 1-6, **dadurch gekennzeichnet, daß** R², R³, R⁴, R^{2'}, R^{3'} und/oder R^{4'} ein 2-Phthalimidoethyl- oder Allyloxy-Rest oder ein Rest der Formel - N[C(O)R⁹]₂ bedeutet.

8. Konjugat nach einem der Ansprüche 1-7, **dadurch gekennzeichnet, daß** das Pentopyranosyl-Nucleosid ein [2',4'-Di-O-Benzoyl-β-ribopyranosyl]-Nucleosid, insbesondere ein [2',4'-Di-O-Benzoyl-β-ribopyranosyl]-adenin, -guanin, -cytosin, - thymidin, -uracil, -xanthin oder Hypoxanthin, ein N-Benzoyl-2',4'-Di-O-benzoylribopyranosyl-Nucleosid, insbesondere ein -adenin, -guanin oder -cytosin, ein N-Isobutyroyl-2',4'-di-O-benzoyl-ribopyranosyl-Nucleosid, insbesondere ein -adenin, - guanin oder -cytosin, ein O⁶-(2-Cyanoethyl)-N²-isobutyroyl-2',4'-di-O-benzoylribopyranosyl-Nucleosid, insbesondere ein -guanin, ein O⁶-(2-(4-Nitrophenyl)ethyl)-N²isobutyroyl-2',4'-di-O-benzoyl-ribopyranosyl-Nucleosid, insbesondere -guanin, ein β-Ribopyranosyl-Nucleoside, insbesonder ein β-Ribopyranosyl-adenin, -guanin, -cytosin, - thymidin oder uracil, xanthin oder hypoxanthin, ein N-Benzoyl-, N-Isobutyroyl-, O⁶-(2-Cyanoethyl)- oder O⁶-(2-(4-Nitrophenyl)ethyl)-N²-isobutyroyl-β-ribopyranosyl-Nucleosid, ein 4'-DMT-pentopyranosyl-Nucleoside, vorzugsweise ein 4'-DMT-ribopyranosyl-adenin, -guanin, -cytosin, -thymidin, -uracil, -xanthin oder hypoxanthin, ein N-Benzoyl-4'-DMT-ribopyranosyl-Nucleosid, insbesondere ein N-Benzoyl-4'-DMTribopyranosyl-adenin, -guanin oder -cytosin, ein N-Isobutyroyl-4'-DMT-ribopyranosyl-Nucleosid, insbesondere ein N-Isobutyroyl-4'-DMT-ribopyranosyl-adenin, -guanin oder - cytosin, ein O⁶-(2-Cyanoethyl)-N²-isobutyroyl-4'-DMT-ribopyranosyl-Nucleosid, insbesondere ein O⁶-(2-Cyanoethyl)-N²-isobutyroyl-4'-DMT-ribopyranosyl-guanin, ein O⁶-(2-(4-Nitrophenyl)ethyl)-N²-isobutyroyl-4'-DMT-ribopyranosyl-Nucleosid insbesondere ein O⁶-(2-(4-Nitrophenyl)ethyl)-N²-isobutyroyl-4'-DMT-ribopyranosylguanin oder ein β-Ribopyranosyl-N,N'-dibenzoyl-adenosin oder ein β-Ribopyranosyl-N,N'-dibenzoyl-guanosin ist.

9. Konjugat nach einem der Ansprüche 1-8, **dadurch gekennzeichnet, daß** das Biomolekül ein Peptid oder Protein ist, dass über einen Aminomodifier, eine Biotingruppe, einen Linker der Formel (IV) oder eine Phthalimidoalkyllinker mit dem Pentopyranosyl-Nucleosid oder mit der Pentopyranosyl-Nucleinsäure verknüpft ist oder dass das Biomolekül eine Ribo- oder Desoxyribo- Nucleinsäure ist, die über eine Phosphatgruppe mit dem Pentopyranosyl-Nucleosid oder mit der Pentopyranosyl-Nucleinsäure verknüpft ist.

10. Konjugat nach gemäß einem der Ansprüche 1-9, **dadurch gekennzeichnet, daß** das Biomolekül ein Antikörper oder ein funktioneller Teil davon oder eine in ihrer natürlichen Form vorkommende DNA und/oder RNA ist.

11. Verfahren zur Herstellung eines Konjugats gemäß einem der Ansprüche 1-10, **dadurch gekennzeichnet, daß** ein Pentopyranosiyl-Nucleosid oder eine Pentopyranosyl-Nucleinsäure mit einem Biomolekül in Verbindung gebracht wird.

12. Verwendung eines Konjugats gemäß einem der Ansprüche 1-10 zur Herstellung eines Arzneimittels oder Diagnostikums.

13. Verwendung eines Konjugats gemäß einem der Ansprüche 1-10 zur Herstellung eines Arrays.

14. Diagnostikum enthaltend ein Konjugat gemäß einem der Ansprüche 1-10.

## Claims

1. Conjugate comprising a pentapyranosylnucleoside or a pentapyranosylnucleic acid and a biomolecule selected from the group consisting of peptides, proteins and nucleic acids.

2. Conjugate according to claim 1, comprising a pentopyranosylnucleoside of the formula (I), 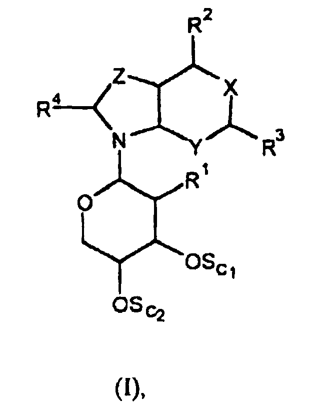 in which
R¹ is equal to H, OH, Hal where Hal is equal to Br or Cl or a radical selected from 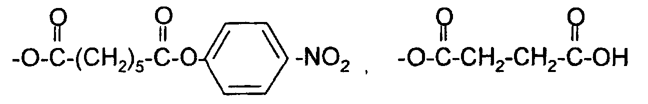 or
-O-P[N(i-Pr)₂]-(OCH₂CH₂CN) where i-Pr is equal to isopropyl, or OS_{c3} where S_{c3} is equal to a protective group, preferably a protective group which can be removed by base or metal catalysis, in particular an acyl group, particularly preferably a benzoyl group,
R², R³ and R⁴ independently of one another, identically or differently, are in each case H, Hal where Hal is equal to Br or Cl, NR⁵R⁶, OR⁷, SR⁸, =O, CₙH₂ₙ₊₁ where n is an integer from 1-12, preferably 1-8, in particular 1-4, a β-eliminable group, preferably a group of the formula -OCH₂CH₂R¹⁸ where R¹⁸ is equal to a cyano or p-nitrophenyl radical or a fluorenylmethyloxycarbonyl (Fmoc) radical, or (CₙH₂ₙ)NR¹⁰R¹¹ where R¹⁰R¹¹ is equal to H, CₙH₂ₙ₊₁ or R¹⁰R¹¹ linked via a radical of the formula 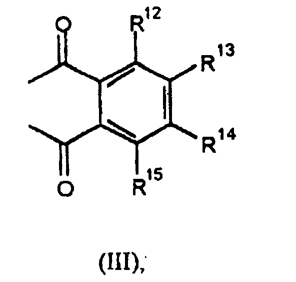 in which R¹², R¹³, R¹⁴ and R¹⁵ independently of one another, identically or differently, are in each case H, OR⁷, where R⁷ has the abovementioned meaning, or CₙH₂ₙ₊₁, or CₙH₂ₙ₋₁, where n has the abovementioned meaning, and
R⁵, R⁶, R⁷ and R⁸ independently of one another, identically or differently, is in each case H, CₙH₂ₙ₊₁, or CₙH₂ₙ₋₁, where n has the abovementioned meaning, -C(O)R⁹ where R⁹ is equal to a linear or branched, optionally substituted alkyl or aryl radical, preferably a phenyl radical,
X, Y and Z independently of one another, identically or differently, is in each case =N-, =C(R¹⁶)- or -N(R¹⁷)-where R¹⁶ and R¹⁷ independently of one another, identically or differently, is in each case H or CₙH₂ₙ₊₁ or (CₙH₂ₙ)NR¹⁰R¹¹ having the abovementioned meanings, and
S_{c1} and S_{c2} independently of one another, identically or differently, is in each case H or a protective group selected from an acyl, trityl or allyloxycarbonyl group, preferably a benzoyl or 4,4'-dimethoxytrityl (DMT) group,
or of the formula (II) 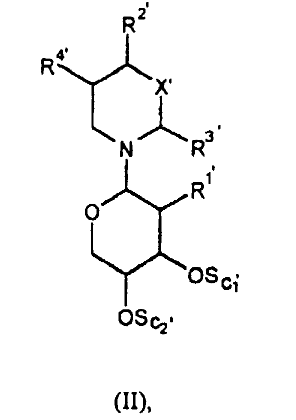 in which R^{1'} is equal to H, OH, Hal where Hal is equal to Br or Cl, or a radical selected from 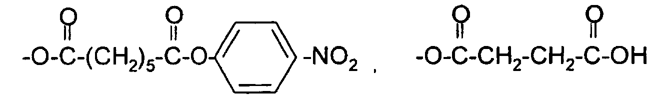 or
-O-P[N(i-Pr)₂]-(OCH₂CH₂CN) where i-Pr is equal to isopropyl, or OS_{c3} where S_{c3} is equal to a protective group, preferably a protective group which can be removed by base or metal catalysis, in particular an acyl group, particularly preferably a benzoyl group,
R^{2'}, R^{3'} and R^{4'} independently of one another, identically or differently, are in each case H, Hal where Hal is equal to Br or Cl, =O, CₙH₂ₙ₊₁ or OCₙH₂ₙ₋₁, a β-eliminable group, preferably a group of the formula -OCH₂CH₂R¹⁸ where R¹⁸ is equal to a cyano or p-nitrophenyl radical or a fluorenylmethyloxycarbonyl (Fmoc) radical, or (CₙH₂ₙ)NR^{10'}R^{11'}, where R^{10'}, R^{11'}, independently of one another has the abovementioned meaning of R¹⁰ or R¹¹, and
X' is =N-, =C(R^{16'})- or -N(R^{17'})-, where R^{16'} and R^{17'} independently of one another have the abovementioned meaning of R¹⁶ or R¹⁷, and S_{c1'} and S_{c2}, have the abovementioned meaning of S_{c1} and S_{c2}.

3. Conjugate according to claim 1 or 2, **characterized in that** an allyloxy linker of the formula
S_{c4}NH(CₙH₂ₙ)CH (OPS_{c5}S_{c6})CₙH₂ₙOS_{c7} (IV)
in which S_{c4} and S_{c7} independently of one another, identically or differently, are in each case a protective group in particular selected from Fmoc and DMT,
S_{c5} and S_{c6} independently of one another, identically or differently, are in each case an allyloxy and/or diisopropylamino group, and n can be an integer from 1 to 12, is incorporated.

4. Conjugate according to one of claims 1-3, **characterized in that** the pentopyranosylnucleoside is a ribo-, arabino-, lyxo- and/or xylopyranosylnucleoside, preferably a ribopyransoylnucleoside.

5. Conjugate according to one of claims 1-4, **characterized in that** the pentopyranosyl moiety of the pentopyranosylnucleoside is in the D or L configuration.

6. Conjugate according to one of claims 1-5, **characterized in that** the pentopyranosylnucleoside employed is a pentopyranosyl purine, -2,6-diaminopurine, -6-purinethiol, -pyridine, -pyrimidine, - adenosine, -guanosine, -isoguanosine, -6-thioguanosine, -xanthine, -hypoxanthine, -thymidine, -cytosine, - isocytosine, -indole, -tryptamine, -N-phthaloyltryptamine, -uracil, -caffeine, -theobromine, - theophylline, -benzotriazole or -acridine.

7. Conjugate according to one of claims 1-6, **characterized in that** R², R³, R⁴, R^{2'}, R^{3'} and/or R^{4'} is a 2-phthalimidoethyl or allyloxy radical or a radical of the formula -N[C(O)R⁹]₂.

8. Conjugate according to one of claims 1-7, **characterized in that** the pentopyranosylnucloside is a [2',4'-di-O-benzoyl-β-ribopyranosyl]nucleoside, in particular a [2',4'-di-O-benzoyl-β-ribopyranosyl]adenine, - guanine, -cytosine, -thymidine, -uracil, -xanthine or hypoxanthine, an N-benzoyl-2',4'-di-O-benzoylribopyranosylnucleoside, in particular an -adenine, - guanine or -cytosine, an N-isobutyroyl-2',4'-di-O-benzoylribopyranosylnucleoside, in particular an - adenine, -guanine or -cytosine, an O⁶-(2-cyanoethyl)-N²-isobutyroyl-2',4'-di-O-benzoylribopyranosylnucleoside, in particular a -guanine, an O⁶-(2-(4'-nitrophenyl)ethyl)-N²-isobutyroyl-2',4'-di-O-benzoyl-ribopyranosylnucleoside, in particular -guanine, a β-ribopyranosylnucleoside, in particular a β-ribopyranosyladenine, -guanine, -cytosine, -thymidine or -uracil, -xanthine or hypoxanthine, an N-benzoyl-, N-isobutyroyl-, O⁶-(2-cyanoethyl)- or O⁶-(2-(4-nitrophenyl)ethyl)-N²-isobutyroyl-β-ribopyranosylnucleoside, a 4'-DMT-pentopyranosylnucleoside, preferably a 4'-DMT-ribopyranosyladenine, -guanine, -cytosine, -thymidine, - uracil, -xanthine or hypoxanthine, an N-benzoyl-4'-DMT-ribopyranosylnucleoside, in particular an N-benzoyl-4'-DMT-ribopyranosyladenine, -guanine or - cytosine, an N-isobutyroyl-4'-DMT-ribopyranosyl-nucleoside, in particular an N-isobutyroyl-4'-DMT-ribopyranosyladenine, -guanine, or -cytosine, an O⁶-(2-cyanoethyl)-N²-isobutyroyl-4'-DMT-ribopyranosyl-nucleoside, in particular an O⁶-(2-cyanoethyl)-N²-isobutyroyl-4'-DMT-ribopyranosylguanine, an O⁶-(2-(-4-nitrophenyl)ethyl)-N²-isobutyroyl-4'-DMT-ribopyranosyl-nucleoside, in particular an O⁶-(2-(-4-nitrophenyl)ethyl)-N²-isobutyroyl-4'-DMT-ribo-pyranosylguanine or a β-ribopyranosyl-N,N'-dibenzoyladenosine or a β-ribopyranosyl-N,N'-dibenzoyl-guanosine.

9. Conjugate according to one of claims 1-8, **characterized in that** the biomolecule is a peptide or protein which is linked via an amino modifier, a biotin group, a linker of the formula (IV) or a phthalimido alkyl linker to the pentopyranosylnucleoside or to the pentopyranosylnucleic acid or **in that** the biomolecule is a ribo- or deoxyribonucleic acid which is linked via a phosphate group to the pentopyranosylnucleoside or to the pentopyranosylnucleic acid.

10. Conjugate according to one of claims 1-9, **characterized in that** the biomolecule is an antibody or a functional moiety thereof or a DNA and/or RNA occurring in its natural form.

11. Process for the preparation of a conjugate according to one of claims 1-10, **characterized in that** a pentopyranosylnucleoside or a pentopyranosylnucleic acid is combined with a biomolecule.

12. Use of a conjugate according to one of claims 1-10 for the production of a medicament or diagnostic.

13. Use of a conjugate according to one of claims 1-10 for the production of an array.

14. Diagnostic comprising a conjugate according to one of claims 1-10.

## Revendications

1. Conjugué contenant un nucléotide pentopyranosyle ou un acide nucléique pentopyranosyle et une biomolécule choisie dans le groupe des peptides, des protéines ou des acides nucléiques.

2. Conjugué selon la revendication 1, contenant un nucléoside pentopyranosyle de formule (I) 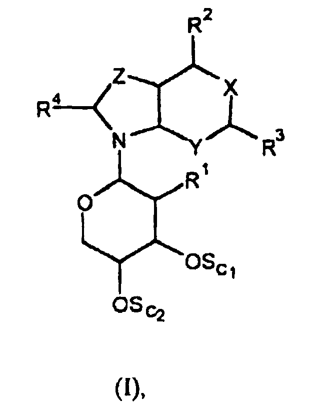 danslaquelle
R¹ représente H, OH, Hal, Hal étant Br ou Cl, ou un reste choisi parmi 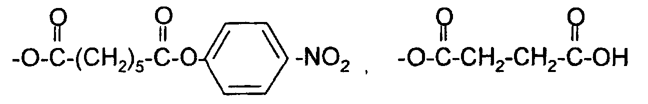 ou
-O-P[N(i-Pr)₂](OCH₂CH₂CN) où i-Pr est un groupe isopropyle, ou OS_{c3}, S_{c3} étant un groupe protecteur, de préférence un groupe protecteur pouvant être séparé de manière catalysée par une base ou un métal, en particulier un groupe acyle, de façon particulièrement préférée un groupe benzoyle,
R², R³ et R⁴ sont identiques ou différents et représentent chacun, indépendamment les uns des autres, H, Hal, Hal étant Br ou Cl, NR⁵R⁶, OR⁷, SR⁸, =O, CₙH₂ₙ₊₁, où n est un nombre entier de 1 à 12, de préférence de 1 à 8, en particulier de 1 à 4, un groupe β-éliminable, de préférence un groupe de formule -OCH₂CH₂R¹⁸ où R¹⁸ est un reste cyano ou p-nitrophényle, ou un reste fluorénylméthyloxycarbonyle (Fmoc), ou un reste (CₙH₂ₙ)NR¹⁰R¹¹ où R¹⁰ et R¹¹ représentent H ou CₙH₂ₙ₊₁ ou R¹⁰ et R¹¹ sont liés par l'intermédiaire d'un reste de formule 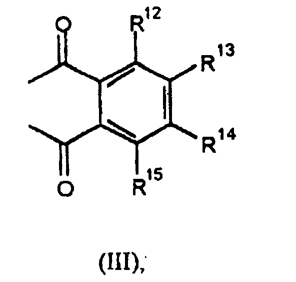 dans laquelle R¹², R¹³, R¹⁴ et R¹⁵ sont identiques ou différents et représentent chacun, indépendamment les uns des autres, H, OR⁷, R⁷ ayant la signification indiquée ci-dessus, ou CₙH₂ₙ₊₁, ou CₙH₂ₙ₋₁, n ayant la signification indiquée ci-dessus, et
R⁵, R⁶, R⁷ et R⁸ sont identiques ou différents et représentent chacun, indépendamment les uns des autres, H, CₙH₂ₙ₊₁, ou CₙH₂ₙ₋₁, n ayant la signification indiquée ci-dessus, -C(O)R⁹ où R⁹ est un reste alkyle linéaire ou ramifié, éventuellement substitué ou aryle, de préférence phényle,
X, Y et Z sont identiques ou différents et représentent chacun, indépendamment les uns des autres, =N-, =C(R¹⁶)- ou -N(R¹⁷)-, où R¹⁶ et R¹⁷ sont identiques ou différents et représentent chacun, indépendamment l'un de l'autre, H ou CₙH₂ₙ₊₁ ou (CₙH₂ₙ)NR¹⁰R¹¹ ayant la signification donnée ci-dessus, et
S_{c1} et S_{c2} sont identiques ou différents et représentent chacun, indépendamment l'un de l'autre, H ou un groupe protecteur choisi parmi les groupes acyle, trityle et allyloxycarbonyle, de préférence un groupe benzoyle ou 4,4'-diméthoxytrityle (DMT),
ou de formule (II) 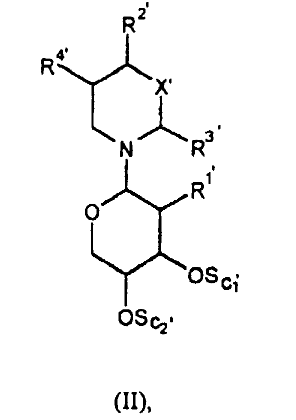 dans laquelle R^{1'} représente H, OH, Hal, Hal étant Br ou Cl, ou un reste choisi parmi 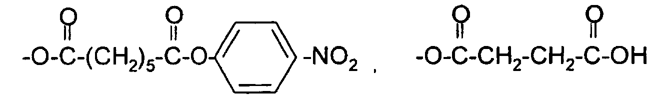 ou
- O-P[N(i-Pr)₂](OCH₂CH₂CN) où i-Pr est un groupe isopropyle, où un groupe OS_{c3}, S_{c3} étant un groupe protecteur, de préférence un groupe protecteur pouvant être séparé de manière catalysée par une base ou un métal, en particulier un groupe acyle, de façon particulièrement préférée un groupe benzoyle,
R^{2'}, R^{3'} et R^{4'} sont identiques ou différents et représentent chacun, indépendamment les uns des autres, H, Hal, Hal étant Br ou Cl, =O, CₙH₂ₙ₊₁ ou CₙH₂ₙ₋₁, un groupe β-éliminable, de préférence un groupe de formule -OCH₂CH₂R¹⁸ où R¹⁸ est un reste cyano ou p-nitrophényle, ou un reste fluorénylméthyloxycarbonyle (Fmoc), ou un reste (CₙH₂ₙ)NR¹⁰'R¹¹' où R^{10'} et R^{11'} ont respectivement, indépendamment l'un de l'autre, la signification de R¹⁰ et R¹¹ indiquée ci-dessus, et
X' représente =N-, =C(R^{16'})- ou -N(R^{17'})-, où R^{16'} et R^{17'} ont respectivement, indépendamment l'un de l'autre, la signification de R¹⁶ et R¹⁷ indiquée ci-dessus, et
S_{c1'} et S_{c2'} ont respectivement, indépendamment l'un de l'autre, la signification de S_{c1} et S_{c2} indiquée ci-dessus.

3. Conjugué selon la revendication 1 ou 2, **caractérisé en ce qu'**est incorporé un lieur allyloxy de formule
S_{c4}NH(CₙH₂ₙ)CH(OPS_{c5}S_{c6})CₙH₂ₙOS_{c7} (IV)
dans laquelle S_{c4} et S_{c7} sont identiques ou différents et représentent chacun, indépendamment l'un de l'autre, un groupe protecteur choisi en particulier parmi les groupes Fmoc et DMT,
S_{c5} et S_{c6} sont identiques ou différents et représentent chacun, indépendamment l'un de l'autre, un groupe allyloxy et/ou diisopropylamino, et n peut être un nombre entier de 1 à 12.

4. Conjugué selon l'une des revendications 1 à 3, **caractérisé en ce que** le nucléoside pentopyranosyle est un nucléoside ribo-, arabino- lyxo- et/ou xylopyranosyle, de préférence un nucléoside ribopyranosyle.

5. Conjugué selon l'une des revendications 1 à 4, **caractérisé en ce que** la partie pentopyranosyle du nucléoside pentopyranosyle a la configuration D ou L.

6. Conjugué selon l'une des revendications 1 à 5, **caractérisé en ce que** l'on utilise comme nucléoside pentopyranosyle un (une) pentopyranosyl-purine, -2,6-diaminopurine, 6-purinethiol, -pyridine, -pyrimidine, -adénosine, -guanosine, - isoguanosine, -6-thioguanosine, -xanthine, -hypoxanthine, -thymidine, -cytosine, - isocytosine, -indole, -tryptamine, -N-phtaloyltryptamine, -uracile, -caféine, - théobromine, -théophylline, -benzotriazole ou -acridine.

7. Conjugué selon l'une des revendications 1 à 6, **caractérisé en ce que** R², R³, R⁴, R^{2'}, R^{3'} et/ou R^{4'} représentent un reste 2-phtalimidoéthyle ou allyloxy ou un reste de formule -N[C(O)R⁹]₂.

8. Conjugué selon l'une des revendications 1 à 7, **caractérisé en ce que** le nucléoside pentopyranosyle est un nucléoside [2',4'-di-O-benzoyl-β-ribopyranosyle], en particulier un (une) [2',4'-di-O-benzoyl-β-ribopyranosyl]-adénine, -guanine, - cytosine, -thymidine, -uracile, -xanthine ou -hypoxanthine, un nucléoside N-benzoyl-2',4'-di-O-benzoylribopyranosyle, en particulier avec l'adénine, la guanine ou la cytosine, un nucléoside N-isobutyroyl-2',4'-di-O-benzoylribopyranosyle, en particulier avec l'adénine, la guanine ou la cytosine, un nucléoside O⁶-(2-cyanoéthyl)-N²-isobutyroyl-2',4'-di-O-benzoylribopyranosyle, en particulier avec la guanine, un O⁶-(2-(4-nitraphényl)éthyl)-N²-isobutyroyl-2',4'-di-O-benzoylribopyranosyle, en particulier avec la guanine, un nucléoside β-ribopyranosyle, en particulier un (une) β-ribopyranosyl-adénine, -guanine, -cytosine, -thymidine ou -uracile, -xanthine ou - hypoxanthine, un nucléoside N-benzoyl-, N-isobutyroyl-, O⁶-(2-cyanoéthyl)- ou O⁶-(2-(4-nitrophényl)éthyl)-N²-isobutyroyl-β-ribopyranosyle, un nucléoside 4'-DMT-pentopyranosyle, de préférence un (une) 4'-DMT-ribopyranosyl-adénine, -guanine, - cytosine, -thymidine, -uracile, -xanthine ou -hypoxanthine, un nucléoside N-benzoyl-4'-DMT-ribopyranosyle, en particulier une N-benzoyl-4'-DMT-ribopyranosyl-adénine, -guanine ou -cytosine, un nucléoside N-isobutyroyl-4'-DMT-ribopyranosyle, en particulier une N-isobutyroyl-4'-DMT-pentopyranosyl-adénine, -guanine ou -cytosine, un nucléoside O⁶-(2-cyanoéthyl)-N²-isobutyroyl-4'-DMT-ribopyranosyle, en particulier une O⁶-(2-cyanoéthyl)-N²-isobutyroyl-4'-DMT-ribopyranosylguanine, un nucléoside O⁶-(2-(4-nitrophényl)éthyl)-N²-isobutyroyl-4'-DMT-nbopyranosyle, en particulier une O⁶-(2-(4-nitrophényl)éthyl)-N²-isobutyroyl-4'-DMT-ribopyranosylguanine, ou une N,N'-dibenzoyladénosine β-ribopyranosyle ou une N,N'-dibenzoylguanosine β-ribopyranosyle.

9. Conjugué selon l'une des revendications 1 à 8, **caractérisé en ce que** la biomolécule est un peptide ou une protéine qui est liée au nucléoside pentopyranosyle ou à l'acide nucléique pentopyranosyle par l'intermédiaire d'un groupe modifiant le groupe amino, d'un groupe biotine, d'un lieur de formule (IV) ou d'un lieur phtalimidoalkyle, ou **en ce que** la biomolécule est un acide ribonucléique ou désoxyribonucléique qui est lié au nucléoside pentopyranosyle ou à l'acide nucléique pentopyranosyle par l'intermédiaire d'un groupe phosphate.

10. Conjugué selon l'une des revendications 1 à 9, **caractérisé en ce que** la biomolécule est un anticorps ou une partie fonctionnelle d'un anticorps ou un ADN et/ou un ARN se présentant sous sa forme naturelle.

11. Procédé de préparation d'un conjugué selon l'une des revendications 1 à 10, **caractérisé en ce que** l'on combine un nucléoside pentopyranosyle ou un acide nucléique pentopyranosyle avec une biomolécule.

12. Utilisation d'un conjugué selon l'une des revendications 1 à 10 pour la préparation d'un médicament ou d'un agent de diagnostic.

13. Utilisation du conjugué selon l'une des revendications 1 à 10 pour la préparation d'une puce.

14. Agent de diagnostic contenant un conjugué selon l'une des revendications 1 à 10.
